(19) Europäisches Patentamt / European Patent Office / Office européen des brevets

(11) **EP 4 679 440 A1**

(12) **EUROPEAN PATENT APPLICATION**

(43) Date of publication:
**14.01.2026 Bulletin 2026/03**

(51) International Patent Classification (IPC):
**G16H 20/17** (2018.01)     **G16H 50/50** (2018.01)
**A61B 5/145** (2006.01)     **A61B 5/00** (2006.01)
**A61M 5/172** (2006.01)

(21) Application number: **25186993.9**

(22) Date of filing: **02.07.2025**

(52) Cooperative Patent Classification (CPC):
**G16H 20/17; A61B 5/4836; A61B 5/4839;
A61B 5/7275; A61M 5/1723; G16H 50/50;**
A61B 5/14532; A61M 2230/201

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.07.2024   US 202463669069 P
09.07.2024   US 202463669067 P
20.05.2025   US 202519214018
20.05.2025   US 202519214017**

(71) Applicant: **Medtronic MiniMed, Inc.
Northridge, CA 91325-1219 (US)**

(72) Inventors:
• **MIKHNO, Arthur**
**Northridge 91325 (US)**
• **ZHONG, Yuxiang**
**Northridge 91325 (US)**
• **STONE, Michael P.**
**Northridge 91325 (US)**
• **CHINCHALKAR, Mihir Satish**
**Northridge 91325 (US)**

(74) Representative: **Zimmermann & Partner
Patentanwälte mbB
P.O. Box 330 920
80069 München (DE)**

(54) **MEAL RESPONSE PREDICTION**

(57)     A processor-implemented method comprises obtaining measured glucose values of a person, fitting a physiological model to a portion of the measured glucose values within a time window after a start of a meal to determine meal-specific values of parameters of the physiological model that characterizes the person's glycemic response to the meal, and predicting a future blood glucose level of the person at a first time after the time window using the physiological model and the meal-specific values of the parameters of the physiological model. In one example, an alert or a notification can be sent to a user or an electronic device based on the predicted future blood glucose level of the person.

FIG. 12

**Description**

CROSS-REFERENCE TO RELATED APPLICATIONS

This application claims the benefit of and priority to U.S. Provisional Application No. 63/669,067, filed July 9, 2024, entitled "RESIDUAL CARB ESTIMATION FOR BOLUS CALCULATION," and U.S. Provisional Application No. 63/669,069, filed July 9, 2024, entitled "MEAL RESPONSE PREDICTION," which are herein incorporated by reference in their entireties for all purposes. The following two U.S. patent applications (including this one) are being filed concurrently, and the entire disclosure of the other application is incorporated by reference into this application for all purposes:

- U.S. Patent Application No. 19/ (Attorney Docket No.: A0009107US02/MEDTP076US), filed , 2025, entitled "RESIDUAL CARB ESTIMATION FOR BOLUS CALCULATION"; and

- U.S. Patent Application No. 19/ (Attorney Docket No.: A0011635US02/MEDTP156US), filed , 2025, entitled "MEAL RESPONSE PREDICTION."

TECHNICAL FIELD

The present disclosure relates generally to blood glucose level management.

BACKGROUND

The pancreas of a healthy person can produce and release insulin into the blood stream in response to elevated blood glucose levels. More specifically, beta cells ($\beta$-cells) in the pancreas can produce and secrete insulin into the blood stream as it is needed. If $\beta$-cells become incapacitated or die, a condition known as Type 1 diabetes mellitus, insulin may need to be provided to the diabetic patient's body using, for example, a syringe, a pen, or an infusion pump of a blood glucose level management system, to maintain life or health.

Some blood glucose level management systems may be closed-loop systems that may include a pump automatically or semi-automatically controlled to deliver insulin to the patient. Some blood glucose level management systems may require manual administration of insulin based on dosage determined by an insulin calculator. The delivery of insulin to the patient can be controlled to occur at times and in amounts that are determined based on, for example, the amount of carbohydrates taken by a patient and/or real-time measurements of glucose levels by a glucose sensor, such as a continuous glucose monitor (CGM). Some blood glucose level management systems may deliver glucose and/or glucagon, in addition to the delivery of insulin, for controlling the blood glucose levels of the patient (e.g., in a hypoglycemic context).

SUMMARY

This disclosure relates generally to blood glucose level management. More specifically, techniques disclosed herein relate to using a physiological model fitted for a person and a specific meal (the type and amount of which may be unknown) to predict the amount of residual carbohydrates in a person's body after the meal so that an appropriate amount of correction bolus can be calculated and delivered to the patient in time to keep the patient's glucose levels within a desired range. The physiological model fitted for the person and the meal can also be used to predict future glucose levels after the meal and, in some embodiments, determine the insulin dose (and/or delivery time) that can keep the patient's future glucose levels within a desired range, based on predicted future glucose levels simulated using the fitted physiological model and acceptable insulin dose. The techniques disclosed herein may be practiced in a variety of ways, such as using a processor-implemented method, a system comprising one or more processors and one or more processor-readable media, and/or one or more (non-transitory) processor-readable media.

According to certain embodiments, the techniques involve obtaining measured glucose values of a person, fitting a physiological model to a portion of the measured glucose values within a time window after a start of a meal to determine meal-specific values of parameters of the physiological model that characterizes the person's glycemic response to the meal, and predicting a future blood glucose level of the person at a first time after the time window using the physiological model and the meal-specific values of the parameters of the physiological model.

This summary is neither intended to identify key or essential features of the claimed subject matter, nor is it intended to be used in isolation to determine the scope of the claimed subject matter. The subject matter should be understood by reference to appropriate portions of the entire specification of this disclosure, any or all drawings, and each claim. The foregoing, together with other features and examples, will be described in more detail below in the following specification, claims, and accompanying drawings.

Further disclosed herein is a processor-implemented method which comprises obtaining measured glucose values of a person, fitting a physiological model to a portion of the measured glucose values within a time window after a start of a meal to determine meal-specific values of parameters of the physiological model that characterizes the person's glycemic response to the meal, and predicting a future blood glucose level of the person at a first time after the time window using the physiological model and the meal-specific values of the parameters of the physiological model. In one example, an alert or a notification can be sent to a user or an electronic device based on the predicted future blood glucose level of the person.

BRIEF DESCRIPTION OF THE DRAWINGS

Illustrative embodiments are described in detail below with reference to the following figures.

FIG. 1A illustrates an example of glucose movement from the blood stream to the interstitial fluid of a person.

FIG. 1B illustrates an example of a function of insulin for facilitating the moving of glucose into cells to provide energy to the cells.

FIG. 1C illustrates examples of glucose generation, movement, and consumption in a person's body.

FIG. 2 illustrates an example of a blood glucose level management system according to certain embodiments.

FIG. 3 is a block diagram of an example of a glucose control system according to certain embodiments.

FIG. 4 includes a diagram illustrating an example of measured sensor glucose (SG) levels of a person before and after a meal.

FIG. 5 illustrates a model showing examples of glucose conversion, transportation, consumption, and measurement in a human body, and insulin delivery, transportation, and consumption in the human body for glucose metabolism according to certain embodiments.

FIG. 6 depicts an example of a delivery device, which may implement some of the examples disclosed herein.

FIG. 7 includes a flowchart illustrating an example of a method of determining appropriate values of parameters of a physiological model associated with a person and a meal for estimating blood glucose levels of the person and/or the dose of a correction bolus after the meal according to certain embodiments.

FIG. 8 illustrates an example of estimating values of parameters of a physiological model associated with a person and a meal according to certain embodiments.

FIG. 9 illustrates another example of estimating values of parameters of a physiological model associated with a person and a meal according to certain embodiments.

FIG. 10 includes a diagram illustrating an example of using a fitted physiological model to estimate sensor glucose levels of a person after a meal and using the physiological model to determine an amount of residual carbohydrates (or residual glucose) and a correction bolus dose according to certain embodiments.

FIG. 11 includes a diagram illustrating another example of using a fitted physiological model to estimate sensor glucose levels of a person after a meal and using the physiological model to determine an amount of residual carbohydrates and a correction bolus dosage according to certain embodiments.

FIG. 12 includes a diagram illustrating yet another example of using a fitted physiological model to estimate sensor glucose levels of a person after a meal and using the physiological model to determine an amount of residual carbohydrates and a correction bolus dosage according to certain embodiments.

FIG. 13 illustrates an example of a process of estimating parameters of a physiological model for a person and a meal and using the physiological model to determine an amount of residual carbohydrates and a correction bolus dosage according to certain embodiments.

FIG. 14 includes a diagram illustrating an example of estimating parameters of a physiological model for a person and a meal and using the physiological model to predict future glucose levels according to certain embodiments.

FIG. 15 includes a diagram illustrating an example of estimating parameters of a physiological model for a person and a meal and using the physiological model to predict future glucose levels and/or determine a correction bolus dosage according to certain embodiments.

FIG. 16 illustrates an example of a process of estimating parameters of a physiological model for a person and a meal and using the physiological model to predict future glucose levels and/or determine a correction bolus dosage according to certain embodiments.

FIG. 17 illustrates an example of a method of estimating parameters of a physiological model for a person and a meal and using the physiological model to predict future glucose levels and/or determine a correction bolus dosage according to certain embodiments.

FIG. 18 is a simplified block diagram of an example of an electronic device that may implement some of the examples disclosed herein.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

This disclosure relates generally to blood glucose (BG) level management. More specifically, techniques disclosed herein relate to using a physiological model fitted for a person and a specific meal (the type and amount of which may be unknown) to predict the amount of residual carbohydrates in a person's body so that an appropriate amount of correction bolus can be calculated and delivered to the patient in time to keep the patient's glucose levels within a desired range. The physiological model fitted for the person and the meal can also be used to predict future glucose levels after the meal and, in some embodiments, may be used to determine the insulin dose (and/or delivery time) that can keep the patient's future glucose levels within a desired range, based on predicted future glucose levels simulated using the fitted physiological model and presumed or potential insulin dose. The techniques disclosed herein may be practiced in a variety of ways, such as using a processor-implemented method, a system comprising one or more processors and one or more processor-readable media, and/or one or more (non-transitory) processor-readable media.

Diabetes mellitus is a disease of the glucose regulatory system of a patient, where the naturally produced insulin in the body may not be sufficient to control the glucose level in the blood stream of the patient, due to insufficient production of insulin and/or insulin resistance. Therefore, a diabetic patient may need to receive insulin from a pump or another delivery device such as an injection or infusion device (e.g., a syringe) to control the glucose level in the patient's blood stream. To control the glucose level, a diabetic patient's therapy routine may generally include dosages of basal insulin and bolus insulin. Basal insulin, also referred to background insulin, may include continuous or constant release of small amounts of insulin to keep blood glucose levels at consistent levels during long time periods. Bolus insulin may be taken specifically before, at, or after mealtimes or other times where there may be a relatively fast rise in glucose level.

The dose of insulin to be delivered may be determined based on, for example, the carbohydrate count of a meal, and/or the glucose level of the patient measured using a glucose monitor, such as a fingerstick blood glucose measurement device or a continuous glucose monitor (CGM). In one example, to counteract an increase in a patient's blood glucose level resultant from the consumption of a meal (or drink), insulin of a certain dose (referred to as a meal bolus) may be delivered to the patient prior to, contemporaneously with, or shortly after the start of the meal. The dose of the insulin may be determined using an insulin calculator (e.g., an App on a user device) that may consider factors such as the intake of carbohydrates, insulin sensitivity factor (ISF) of the patient, and the patient's physiological condition, and may indicate the number of units of insulin to be delivered. Too much insulin can lead to hypoglycemia, while too little insulin can lead to hyperglycemia.

Existing insulin calculators may generally use the carbohydrate entry for bolus recommendation. This may work reasonably well for meal rich in carbohydrates. In many cases, a meal may also include macronutrients such as fat and protein, which may affect the conversion of the meal into glucose and the absorption of glucose. For example, proteins may be converted into amino acids and absorbed into the blood stream, whereas fats and triglycerides may be absorbed in lymph system and enter blood stream via lymph vessels. In addition, meals containing mainly carbohydrates may be absorbed at different rates. For example, meals containing fast-digesting carbohydrates (or high glycemic index carbohydrates), such as white bread, soda drinks, honey, donuts, cakes, white rice, cereals, watermelon, and the like, may be digested and absorbed at faster rates. Therefore, some meals or drinks (e.g., including carbohydrates with high glycemic indices) may be converted and absorbed faster than other meals or drinks (e.g., carbohydrates including medium or low glycemic indices). As such, the meal composition may affect a person's glycemic response. Furthermore, in some circumstances, the patient may forget to log some meals, and thus information about past meals may not be available or may not be accurate for determining the bolus dose. Additionally, the patient's individual carbohydrate to glucose conversion factor, carbohydrate absorption rate, insulin absorption factor, insulin response (e.g., insulin sensitivity), and the like may vary over time and may be affected by, for example, daily activities (e.g., exercise), stress, circadian rhythms, time of the day, the environment (e.g., temperature), and the like. As such, in many cases, a meal bolus calculated based on the amount of carbohydrate intake may not be accurate or adequate, and thus may sometimes fail to keep the blood glucose level within the desired range.

To address this problem, one or more correction boluses may need to be delivered, for example, before the BG level reaches a high level due to insufficient meal bolus. However, correction boluses are typically reactive and difficult to precisely calculate, and thus may result in undesirable swings in glucose levels. In some implementations, the dose of the correction bolus may be determined based on the current BG level (e.g., sensor glucose (SG) value measured by a CGM), a target BG level, the amount of active insulin (e.g., insulin on board (IOB)), and the ISF. However, the dose of the correction bolus calculated based on the difference between the current BG level and the target BG level may not be accurate due to, for example, residual carbohydrates that may still exist in patient's body and may continue to contribute to the increase in the BG level.

According to certain embodiments, after a meal is detected or logged, parameters associated with the patient's physiological response (e.g., glycemic response) to the meal, which may be parameters of a physiological model for the patient, may be determined, for example, by fitting the physiological model to glucose data measured during a certain time period (e.g., within a meal response period or a fitting time period) after the meal. The physiological model fitted using the measured glycemic response of the patient body to the specific meal may more accurately reflect the patient's glycemic response to the specific meal, including the type and amount of food and/or the condition of the meal (e.g., the time of the day, the user's activity and/or mental state, weather, temperature, etc.). The physiological model with the determined meal-specific parameters can be used to predict the total amount of glucose level increase that may result from the meal. The total amount of glucose level decrease that may result from active insulin (if any) after the start of the meal may also be determined using an insulin model or the same physiological model (which may be a unified model). Therefore, the amount of residual glucose (the amount of glucose remaining as a result of the amount of glucose level decrease due to active insulin and the amount of glucose level increase due to the meal) may be determined using the fitted physiological model. The amount of residual glucose may be converted into an amount of residual carbohydrates. The amount of residual glucose or residual carbohydrates may be used to determine the dose of a correction bolus (also referred to as the remedial dose or supplemental dose) for the patient, before the patient's blood glucose level may otherwise reach a high level or a postprandial settling value, such that the correction bolus may be delivered to the patient early to keep the blood glucose level within the desired range. As a result, the number of correction boluses needed for a meal may be reduced, and the clinical outcomes (e.g., time in range) may be improved.

In some embodiments, based on the physiological model with the determined meal-specific model parameters and the correction bolus administered (if any), future glucose levels of the patient may be predicted. In some embodiments, the physiological model with the meal-specific model parameters may be used to determine the appropriate insulin delivery dose/timing to keep the patient's future glucose levels within the desired range. For example, the insulin calculator may calculate an initial correction insulin dose (and determine the delivery time), and the physiological model may be used to predict future glucose levels based on the initial correction insulin dose. The initial correction insulin dose (and/or the delivery time) may be adjusted based on the predicted future glucose levels (e.g., whether the glucose levels may be out of range). The correction insulin dose that can achieve the desired future glucose levels according to the prediction using the physiological model may be selected and administered to the patient.

Aspects and embodiments of the present disclosure can be practiced with one or more types of insulin (e.g., fast-acting insulin, intermediate-acting insulin, and/or slow-acting insulin). Unless indicated otherwise by the context, terms such as "dose," "insulin," "basal," and "bolus" may not denote a particular type of insulin. For example, fast-acting insulin may be used for both basal dosages and bolus dosages. The term "basal" can refer to and include insulin that is delivered in an amount and at a frequency that is intended to correspond to a healthy body's release of insulin between meals and during sleep. The term "bolus" may refer to and include insulin that is delivered in an amount and at a timing that is intended to correspond to a healthy body's release of insulin for counteracting a high glucose level, such as that caused by the consumption of a meal. A meal may include any type or amount of food or beverage consumption, including breakfast, lunch, dinner, snacks, and beverages, among others.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, operations, and techniques may be shown without necessary detail or may not be shown, in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

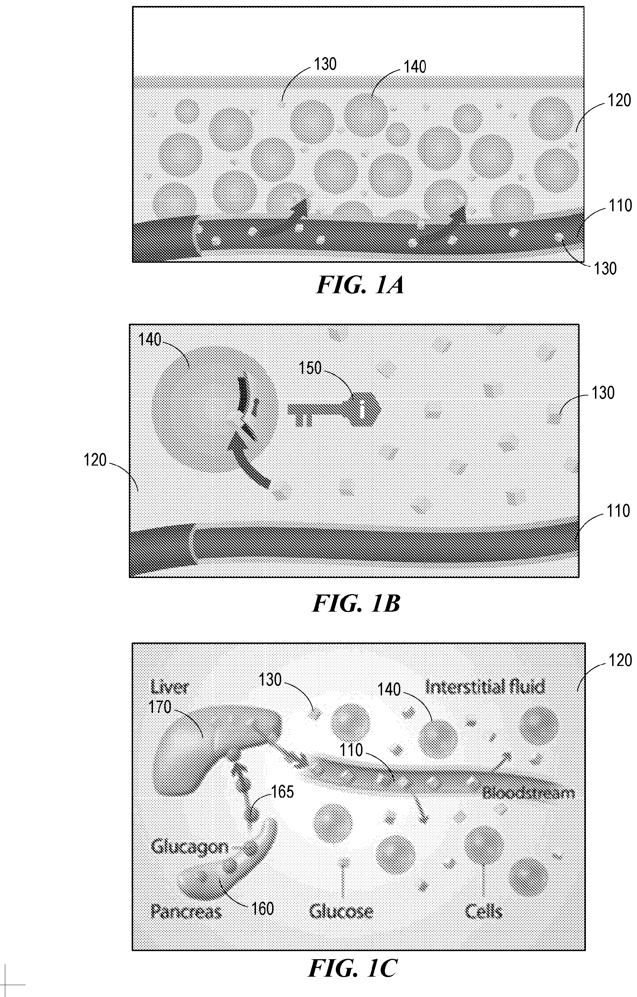

FIG. 1A illustrates an example of glucose movement from a blood stream 110 to the interstitial fluid 120 of a person. Carbohydrates in food intake may be broken down into glucose (sugar) in the stomach and/or intestine, and may be absorbed at the small intestine and/or large intestine into blood stream 110. Blood stream 110 may transport glucose 130 to the capillaries of the body, where some glucose 130 may move into interstitial fluid 120 between cells 140 (e.g., fat or muscle cells), which may use glucose 130 for energy.

The endocrine system of the human body that directs and regulates the functions and activities of the body (works with a nervous system) may secrete chemicals that transmit messages to tissues and organs. For example, endocrine glands or organs may release hormones into the blood stream to target cells with receptors. In one example, insulin may be made by the β-cells of pancreas, and may, for example, increase the glucose uptake, enhance glucose utilization, stop hepatic glucose production, stimulate glycogen formation in liver and skeletal muscle, promote protein synthesis, and increase fat storage.

FIG. 1B illustrates an example of a function of insulin 150 for facilitating the moving of glucose 130 into cells 140 to provide energy to cells 140. Insulin 150 may function as a key that can unlock cells 140 and help glucose 130 to move into cells 140 where glucose 130 may be used for energy. Without insulin 150, glucose may not be able to enter cellsc140 to be used for energy, and may build up in the interstitial fluid and blood stream. A healthy pancreas may continuously release a small amount of regular human insulin 24 hours a day, including between meals and during sleep. The small amount pf insulin may match the liver's release of glucose. A healthy pancreas may also secrete a larger amount of insulin after food intake to match the amount of food intake.

FIG. 1C illustrates examples of glucose generation, movement, and consumption in a person's body. A liver 170 of the body may absorb excessive glucose from digestion and convert it to glycogen for storage so that glucose may be available when needed. For example, when the blood glucose level is low, the α-cells of pancreas 160 may secrete glucagon 165. Glucagon 165 may cause liver 170 to release the stored form of glucose (glycogen) into blood stream 110 to help increase the glucose level. The balance between insulin secreted by the β-cells and the glucagon secreted by the α-cells may help to maintain the normal blood glucose level, such as in the range of about 80-120 mg/dL before meals.

The naturally produced insulin in the body of a diabetic patient may not be sufficient to control the glucose level in the blood stream of the patient, due to insufficient production of insulin and/or insulin resistance. Therefore, a diabetic patient may need to receive insulin from a pump or another delivery device such as an injection or infusion device to control the glucose level in the patient's blood stream. To control the glucose level, a diabetic patient's therapy routine may generally include dosages of basal insulin and bolus insulin. Dosages of insulin to be delivered may be determined based on, for example, the carbohydrate count of a meal, and/or the glucose level of the patient measured using a glucose monitor, such as a continuous glucose monitor (CGM). In one example, to counteract an increase in a patient's blood glucose level resultant from the consumption of a meal (or drink), insulin of a certain dose (often referred to as a meal bolus) may be delivered to the patient prior to, contemporaneously with, or shortly after the start of the meal. The dose of the insulin may be determined using an insulin calculator (e.g., an App on a user device) that may consider factors such as the intake of carbohydrates and insulin sensitivity factor (ISF) of the patient. The insulin may be delivered to the diabetic patient's interstitial fluid using, for example, an insulin delivery device including a percutaneous cannula configured to deliver insulin to the patient, or a hand-held injection device, such as a syringe, a smart insulin pen, or a disposable insulin pen.

In some implementations (e.g., in a closed-loop system), the insulin delivery device may communicate with or otherwise use a sensor device (including but not limited to a CGM) to perform various measurements for a patient. In one example, the sensor device may include subcutaneous implanted electrodes to concurrently monitor the patient's response to meals and insulin introduced by the insulin delivery device. The sensor device and the insulin delivery device may be in a communication network (wired or wireless) with one or more processors and/or patient devices (such as a patient's smartphone equipped with an application or other software) to create an overall system for monitoring a patient disease state and for facilitating treatment thereof.

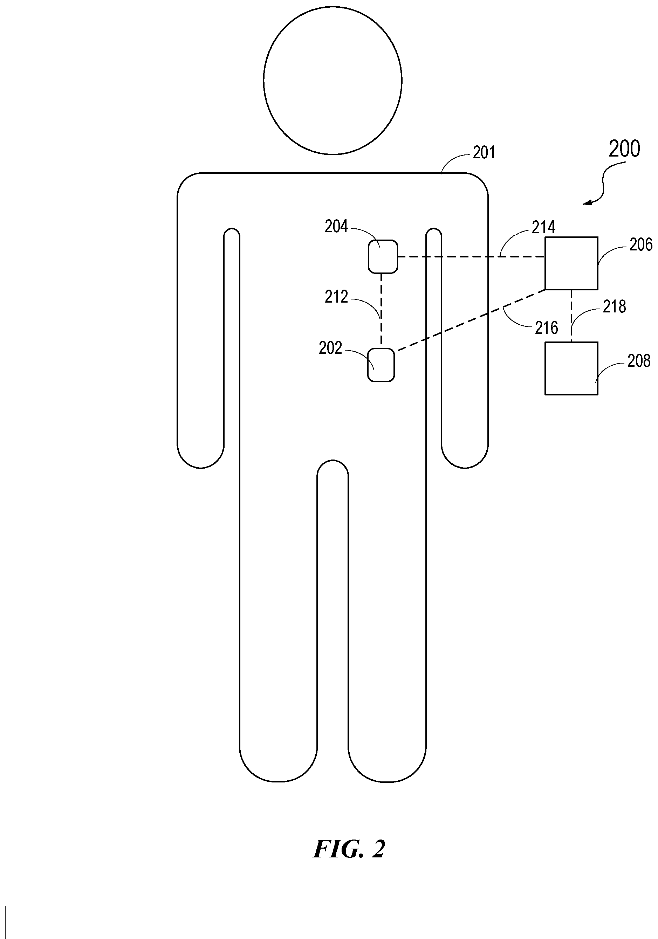

FIG. 2 illustrates an example of a blood glucose level management system 200 according to certain embodiments. Blood glucose level management system 200 may be used to monitor and regulate the blood glucose level of a patient 201. In the illustrated example, blood glucose level management system 200 may include a delivery device 202, a monitoring device 204, a computing device 206, and an optional remote/cloud computing system 208. Delivery device 202, monitoring device 204, and computing device 206 may be embodied in various ways, including being disposed in one or more device housings. For example, in some embodiments, all of devices 202-206 may be disposed in a single device housing. In some embodiments, each of devices 202-206 may be disposed in a separate device housing. In some embodiments, two or more of devices 202-206 may be disposed in the same device housing. In some embodiments, a single device 202, 204, or 206 may have two or more parts that are disposed in two or more housings. For example, monitoring device 204 may include an on-body part and a display and control part communicated with the on-body part through wires or wirelessly. Delivery device 202 may include an on-body site (e.g., including a cannula) and a part that includes a reservoir, a pump, and a control unit. These and other embodiments, and combinations thereof, are contemplated to be within the scope of the present disclosure.

Blood glucose level management system 200 may include a plurality of communication links, such as communication links 212-218. Communications links 212-1418 may each be a wired connection and/or a wireless connection. In embodiments where two devices are located in a same housing, the communication link may include, for example, wires, cables, and/or communication buses on a printed circuit board, among other things. In embodiments where two devices are separate from each other in different device housings, the communication links may be wired and/or wireless connections. Wired connections may include, for example, an Ethernet connection, a Universal Serial Bus (USB) connection, and/or another type of physical connection. Wireless connections may include, for example, a cellular connection, a Wi-Fi connection, a Bluetooth® connection, a mesh network connection, and/or another type of connection using a wireless communication protocol. Some embodiments of communication links 212-218 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for communication links 212-218.

Delivery device 202 may be configured to deliver a therapeutic substance to patient 201. The therapeutic substance may include, for example, insulin, HIV drugs, drugs to treat pulmonary hypertension, iron chelation drugs, pain medications, anti-cancer treatments, medications, vitamins, hormones, a nutritional supplement, a dye, a tracing medium, a saline medium, a hydration medium, and the like. Delivery device 202 may be secured to patient 201 (e.g., to the body or clothing of patient 201) or may be at least partially implanted in the body of patient 201. In some embodiments, the delivery device 202 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of patient 201. The cannula and/or a needle may facilitate delivery of the therapeutic substance to a tissue layer, vein, interstitial fluid, or body cavity of patient 201. During operation, the actuator, in response to a signal (e.g., a command signal), may drive the delivery mechanism, thereby causing the therapeutic substance to move from the reservoir, through the cannula, and into the body of patient 201.

The components of delivery device 202 described above are merely provided as an example. Delivery device 202 may include other components, such as, without limitation, a power supply, a communication transceiver, one or more processors or other computing resources, memory devices, and/or user interfaces (e.g., buttons, keys, display, etc.), among other things. In some implementations, delivery device 202 may host an App (e.g., an insulin calculator) that may calculate the desired amount of therapeutic substance to be delivered to patient 201. Persons skilled in the art will recognize various implementations of delivery device 202 and the components of such implementations. All such implementations and components are contemplated to be within the scope of the present disclosure.

Monitoring device 204 may be configured to detect a physiological condition (e.g., a glucose concentration level) of patient 201 and may also be configured to detect other things. Monitoring device 204 may be secured to the body of patient 201 (e.g., to the skin of patient 201 via an adhesive) and/or may be at least partially implanted into the body of patient 201. Depending on the particular location or configuration, monitoring device 204 may be in contact with biological matter (e.g., interstitial fluid and/or blood) of patient 201.

Monitoring device 204 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode. When a voltage signal is supplied, the electrical charges may be forced to move, thereby generating a sensor current signal (Isig) that can be measured by sensor electronics. Other signals such as the counter electrode voltage (Vcntr), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

As persons skilled in the art would understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of patient 201. In some embodiments, the electrical biosignal may include portions of a potential change produced by the heart of patient 201 over time (e.g., recorded as an electrocardiogram) that may be indicative of a glucose level of patient 201. An optical sensor may be configured to, for example, respond to the interaction or binding of a biological marker to a substrate by generating an electrical signal based on change in luminance of the substrate. In one example, the substrate may include a material selected to fluoresce in response to contact with a selected biomarker, such as glucose. The fluorescence may be proportional to a concentration of the selected biomarker.

In some embodiments, monitoring device 204 may include other types of sensors that may be worn, carried, or coupled to patient 201 to measure activity of patient 201 that may influence the glucose levels or glycemic response of patient 201. As an example, the sensors may include an acceleration sensor configured to detect an acceleration of patient 201 or a portion of the patient 201, such as the person's hands or feet, the position changes of which may be associated with an activity of patient 201. For example, the acceleration or movement (or lack thereof) of the body portion of patient 201 may be indicative of exercise, sleep, or food/beverage consumption activity of patient 201, which may influence the glycemic response of patient 201. In some embodiments, the sensors may measure heart rate and/or body temperature, which may indicate an amount of physical exertion experienced by patient 201. In some embodiments, the sensors may include a Global Positioning System (GPS) receiver which may detect GPS signals to determine a location of patient 201.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor may be referred to herein as a "sensor signal." As used herein, the term "sensed data" may mean and include the information represented by a sensor signal or by a pre-processed sensor signal. In some embodiments, sensed data may include glucose levels of patient 201, acceleration of a part of patient 201, heart rate of patient 201, temperature of patient 201, and/or geolocation (e.g., GPS location) of patient 201, among other things. Monitoring device 204 may communicate sensed data to delivery device 202 via communication link 212 and/or to computing device 206 via communication link 214. Use of sensed data by delivery device 202 and/or by computing device 206 is described in more detail below.

In some embodiments, monitoring device 204 may include components and/or circuitry configured to pre-process sensor signals. Pre-processing may include, for example, amplification, filtering, attenuation, scaling, isolation, normalization, transformation, sampling, and/or analog-to-digital conversion, among other things. In some embodiments, monitoring device 204 may host an App for processing the sensor signals. In some embodiments, monitoring device 204 may include a wired or wireless transceiver as described above for transmitting the sensor signals or receiving commands or instructions. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, monitoring device 204 may not perform pre-processing.

Computing device 206 may provide processing capabilities and may be implemented in various ways. In some embodiments, computing device 206 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of delivery device 202. In some embodiments, computing device 206 may be processing circuitry that may be integrated with another device, such as delivery device 202. In some embodiments, computing device 206 may be secured to patient 201 (e.g., to the body or clothing of patient 201), may be at least partially implanted into the body of patient 201, and/or may be held by patient 201.

For each of the embodiments of computing device 206, computing device 206 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Some aspects of delivery device 202, monitoring device 204, and computing device 206 have been described above. One or more of devices 202-206 may include a user interface (not shown) that presents information to patient 201 and/or receives information from patient 201. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where computing device 206 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify patient 201 of sensed data (e.g., glucose level) and/or insulin delivery data (e.g., rates of historic, current, or future insulin delivery) and may present alerts to patient 201. In some embodiments, a user interface may receive inputs from patient 201, which may include, for example, a requested change in insulin delivery setting and/or a meal indication, among other things. The descriptions and embodiments above regarding user interfaces are merely provided as examples, and other types and other uses of user interfaces are contemplated to be within the scope of the present disclosure.

In one specific example, the communications between devices 202-206 and cooperation between devices 202-206 may be used for insulin delivery. As depicted in FIG. 2 and as described above, devices 202-206 may communicate with each other via communication links 212-216. In some embodiments, computing device 206 may control operations of delivery device 202 and/or monitoring device 204. For example, computing device 206 may generate one or more signals (e.g., a command signal) that cause delivery device 202 to deliver insulin to patient 201, for example, as a basal dosage and/or a bolus dosage. In some embodiments, computing device 206 may receive data associated with insulin delivery (e.g., insulin delivery data) from delivery device 202 and/or receive sensed data (e.g., glucose levels) from monitoring device 204, and may perform computations based on the insulin delivery data, the sensed data, and/or other data to control delivery device 202. Insulin delivery data may include, but is not limited to, the type of insulin being delivered, historical insulin delivery rates and/or amounts, current insulin delivery rate and/or amount, insulin delivery time, and/or user inputs affecting insulin delivery. As persons skilled in the art will understand, in a closed-loop operating mode, computing device 206 may communicate dosage commands to delivery device 202 based on, for example, a difference between a current glucose level in the body of patient 201 (e.g., received from monitoring device 204) and a target glucose level (e.g., determined by computing device 206 or set on delivery device 202). The dosage commands may indicate an amount of insulin to be delivered and/or a rate (or time) of insulin delivery, and may regulate the current glucose level toward the target glucose level.

Remote/cloud computing system 208 may be any proprietary remote/cloud computing system or a commercial cloud computing system including one or more server computing devices. Remote/cloud computing system 208 may provide alternative or additional computing resources as needed when the computing resources of a client computing device (e.g., computing device 206) are not sufficient. Computing device 206 and remote/cloud computing system 208 may communicate with each other through a communication link 218, which may traverse one or more communication networks (not shown). The communication networks may include, for example, an Ethernet network, a Wi-Fi network, a cellular network, a satellite network, an intranet, an extranet, the Internet, and/or the Internet backbone, among other types of networks. Persons skilled in the art will recognize implementations for remote/cloud computing system 208 and how to interface with such systems through various types of networks. For example, remote/cloud computing system 208 may include an array of processing circuitry and may execute machine-readable instructions. Such implementations, interfaces, and networks are contemplated to be within the scope of the present disclosure.

In some embodiments, remote/cloud computing system 208 may make a therapy determination (e.g., an insulin amount or adjusted insulin amount), and may communicate the therapy to delivery device 202 via computing device 206. In some embodiments, computing device 206 may make the therapy determination and communicate it to delivery device 202. In some embodiments, monitoring device 204 may make the therapy determination and communicate it to delivery device 202 either directly or through an intermediary such as computing device 206.

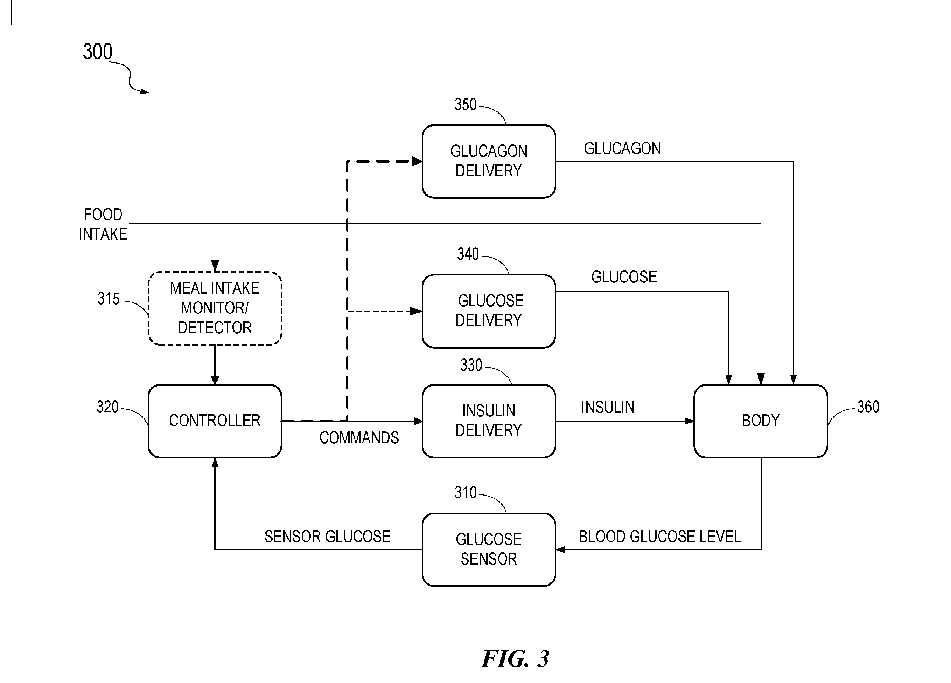

FIG. 3 is a block diagram of an example of a glucose control system 300 according to certain embodiments. In the illustrated example, glucose control system 300 may include a glucose sensor subsystem 310, a controller 320, an insulin delivery subsystem 330, a glucose delivery subsystem 340, and a glucagon delivery subsystem 350. Glucose sensor subsystem 310 may generate sensor glucose (SG) signals (e.g., SG levels) that may be the estimations of blood glucose levels in a body 360, and may provide the SG signals to controller 320. Controller 320 may receive the SG signals and generate commands to insulin delivery subsystem 330, and, in some implementations, glucose delivery subsystem 340 and/or glucagon delivery subsystem 350. Insulin delivery subsystem 330 may receive commands from controller 320 and deliver insulin to body 360 according to the commands. In some embodiments, glucose delivery subsystem 340 may receive commands from controller 320 and provide glucose into body 360 according to the commands. In some embodiments, glucagon delivery subsystem 350 may receive commands from controller 320 and deliver glucagon into body 360 according to the commands.

In some implementations, glucose sensor subsystem 310 may include a glucose sensor, sensor electronics configured to generate SG signals, a sensor communication system configured to send the SG signals to controller 320, and a housing for the sensor electronics and the sensor communication system. The glucose sensor may measure blood glucose levels, for example, directly from a blood stream, or indirectly via interstitial fluid using a subcutaneous sensor.

Controller 320 may include electrical components and software to generate commands for insulin delivery subsystem 330, glucose delivery subsystem 340, and/or glucagon delivery subsystem 350. Controller 320 may include a controller communication system to receive the sensor signal and provide the commands to insulin delivery subsystem 330, glucose delivery subsystem 340, and/or glucagon delivery subsystem 350. In some implementations, controller 320 may implement a glucose calculator. In some implementations, controller 320 may include a user interface and/or operator interface (not shown) comprising a data input device and/or a data output device. Such a data output device may, for example, generate signals to initiate an alarm and/or include a display or printer for showing status of controller 320 and/or a patient's vital indicators. Such a data input device may include dials, buttons, pointing devices, manual switches, alphanumeric keys, a touch-sensitive display, combinations thereof, and/or the like for receiving user and/or operator inputs. Such a data input device may be used for scheduling and/or initiating insulin bolus injections for meals, for example. It should be understood, however, that these are merely examples of input and output devices that may be a part of an operator and/or user interface and that claimed subject matter is not limited in these respects.

Insulin delivery subsystem 330 may include, for example, an infusion device and/or an infusion tube to infuse insulin into body 360. Similarly, glucose delivery subsystem 340 may include, for example, an infusion device and/or an infusion tube to infuse glucose into body 360. Likewise, glucagon delivery subsystem 350 may include, for example, an infusion device and/or an infusion tube to infuse glucagon into body 360. In some embodiments, the insulin, glucagon, and/or glucose may be infused into body 360 using a shared delivery system and/or infusion tube. In some embodiments, the insulin, glucagon, and/or glucose may be infused using an intravenous system for providing fluids to a patient (e.g., in a hospital or other medical environment). It should be understood, however, that certain example embodiments may include an insulin delivery subsystem 330 without a glucagon delivery subsystem 350 and/or without a glucose delivery subsystem 340. In some embodiments, each of insulin delivery subsystem 330, glucose delivery subsystem 340, and glucagon delivery subsystem 350 may include infusion electrical components to activate an infusion motor according to the commands from controller 320, an infusion communication system to receive commands from controller 320, and a delivery subsystem housing.

In some embodiments, controller 320 may be housed in a delivery subsystem housing, and an infusion communication system may comprise an electrical trace or a wire that carries the commands from controller 320 to the delivery subsystem. In some embodiments, controller 320 may be housed in a sensor system housing, and a sensor communication system may comprise an electrical trace or a wire that carries the sensor signal from sensor electrical components to controller electrical components. In some embodiments, controller 320 may have its own housing or may be included in a supplemental device. In some embodiments, controller 320 may be co-located with a delivery subsystem and a sensor system within a single housing. In some embodiments, a sensor, a controller, and/or infusion communication systems may utilize a cable; a wire; a fiber optic line; RF, IR, or ultrasonic transmitters and receivers; combinations thereof; and/or the like instead of electrical traces, just to name a few examples.

In some embodiments, glucose control system 300 may also include a meal intake monitoring subsystem 315. Meal intake monitoring subsystem 315 may be used to log the amount of user food intake, or may automatically detect the amount of user food intake. For example, in some implementations, the user may enter the food items and/or the estimated amount of carbohydrates in a meal at the meal time. In some implementations, meal intake monitoring subsystem 315 may include sensors (e.g., cameras or accelerators) that may automatically detect a meal event, the food items in a meal, and/or the estimated amount of carbohydrates in the meal. The estimated amount of carbohydrates in the meal may be sent to controller 320, which may determine an appropriate amount of meal bolus and generate a command for insulin delivery subsystem 330 to deliver the meal bolus. In some implementations, meal intake monitoring subsystem 315 may not be used in glucose control system 300, and the dosage for the meal bolus may be determined based on the measured glucose level.

As described above, the dose of the insulin delivered to a patient may be determined using an insulin calculator (e.g., an App on a user device, such as computing device 206 or controller 320) that may consider factors such as the intake of carbohydrates, insulin sensitivity factor (ISF) of the patient, and the patient's physiological condition (e.g., measured sensor or blood glucose level, etc.), and may indicate the number of units of insulin to be delivered. Too much insulin may lead to hypoglycemia, whereas too little insulin may lead to hyperglycemia.

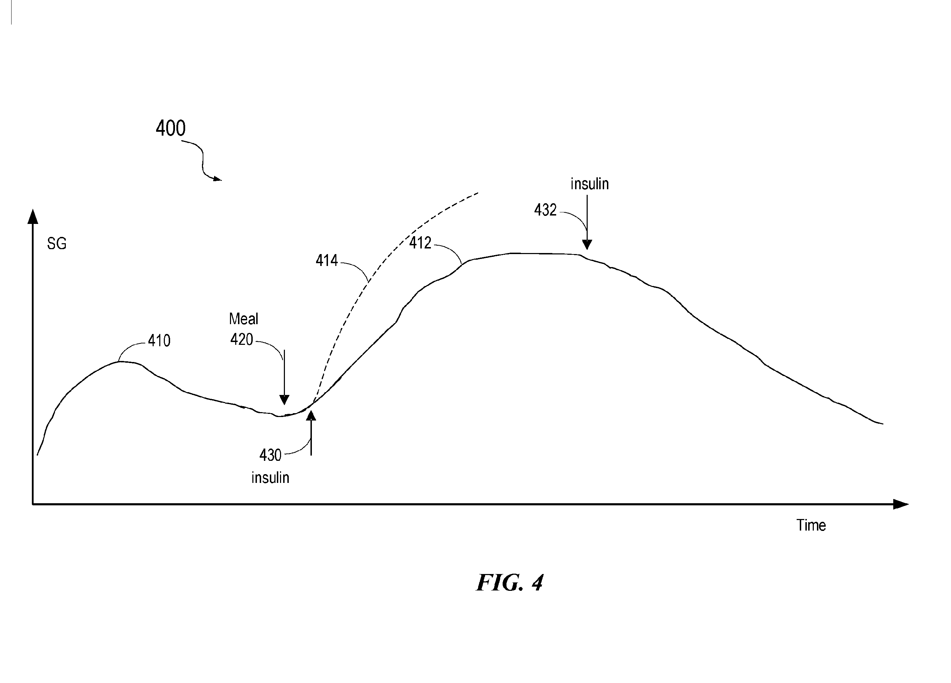

FIG. 4 includes a diagram 400 illustrating an example of measured sensor glucose (SG) levels of a person before and after a meal. The SG levels may be measured using, for example, glucose sensor subsystem 310, such as a CGM. The measured time series of SG levels as a function of time is shown by a curve 410. As described above, small dosages of basal bolus may be continuously delivered to the patient to maintain the blood glucose level of the patient within a certain range. When a meal 420 is taken by the patient, if no additional insulin is delivered, the blood glucose level of the patient may increase quickly as shown by a curve 414. To counter the glucose level increase caused by the meal, a meal bolus 430 may need to be delivered to the patient to prevent the blood glucose level from reaching a level that may cause hyperglycemia. Meal bolus 430 may be delivered before, during, or after the start of a meal. The dose of meal bolus 430 may be determined according to:

$$B = \frac{carb}{ICR} + \frac{(BG - Target)}{ISF} - IOB, \qquad (1)$$

where $B$ is the number of units of insulin of the meal bolus to be delivered, $carb$ is the amount of carbohydrate intake, $ICR$ is the patient specific insulin-to-carbohydrate ratio (ICR) (e.g., grams of carbohydrates covered by each insulin unit), $BG$ is the current blood glucose level, $target$ is the target blood glucose level (e.g., a value between about 70-180 milligrams per deciliter (mg/dL)), $ISF$ is the patient specific insulin sensitivity factor (e.g., the amount (in mg/dL) of glucose level reduction by each insulin unit), and $IOB$ is the insulin on board (e.g., active or unmetabolized insulin in the patient's body). A portion 412 of curve 410 shows the measured SG levels after the intake of meal 420 and the delivery of meal bolus 430.

In many cases, the dose of meal bolus 430 determined using Equation 1 may not be sufficient to cover the meal due to, for example, the type of food that may impact the carbohydrate absorption, the variation of the patient's $ICR$, the variations of other glycemic response parameters of the patient, and the like. For example, as described above, meals containing carbohydrates having different glycemic indices may be absorbed at different rates, and thus the blood glucose level of the patient may increase at different rates, even if the different meals have the same amount of carbohydrates. In addition, in many cases, a meal may include macronutrients such as fat and protein, which may also affect the conversion of carbohydrates into glucose and the absorption of glucose. Proteins may be converted into amino acids and absorbed into the blood stream, and fats and triglycerides may be absorbed in lymph system and enter blood stream via lymph vessels. Therefore, some meals or drinks may be converted and absorbed faster than other meals or drinks. As such, the meal composition may affect a person's glycemic response. Furthermore, in some circumstances, the patient may forget to log some meals, and thus information about past meals may not be available or may not be accurate for determining the bolus dose. Additionally, the patient's individual carbohydrate to glucose conversion factor, carbohydrate absorption rate, insulin absorption factor, insulin response (e.g., insulin sensitivity), and the like may vary over time and may be affected by, for example, daily activities (e.g., exercise), stress, circadian rhythms, time of the day, the environment (e.g., temperature), and the like. As such, in many cases, a meal bolus calculated based on the amount of carbohydrate intake may not be accurate or adequate, and thus may sometimes fail to keep the blood glucose level within the desired range.

To address this problem, one or more correction boluses 432 may be delivered, for example, before the BG level reaches a high level due to insufficient meal bolus. Correction boluses are typically reactive and difficult to precisely calculate, and thus may result in undesirable swings in glucose levels. In some implementations, the dose B of a correction bolus 432 may be determined based on the current blood glucose level $BG$ (e.g., SG value measured by a CGM), the target BG level $target,$ the amount of active insulin (e.g., the insulin on board $IOB),$ and the patient's insulin sensitivity factor $ISF$ according to:

$$B = \frac{(BG - Target)}{ISF} - IOB. \qquad (2)$$

The dose of the correction bolus calculated based on the difference between the current BG level and the target BG level according to Equation 2 may not be accurate due to, for example, residual carbohydrates that may still exist in patient's body and may continue to contribute to an increase in the BG level.

According to certain embodiments, after a meal is detected or logged, parameters associated with the patient's physiological response (e.g., glycemic response) to the meal, which may be parameters of a physiological model for the patient, may be determined, for example, by fitting the physiological model to glucose data measured during a certain time period (e.g., within a meal response period or a fitting time period) after the meal. The physiological model fitted using the measured glycemic response of the patient body to the specific meal may more accurately reflect the patient's glycemic response to the specific type of food under the current condition (e.g., the time of the day, the user's activity and/or mental state, weather, temperature, etc.). The physiological model with the determined meal-specific parameters can be used to predict the total amount of glucose level increase that may result from the meal. The total amount of glucose level decrease that may result from active insulin (if any) after the start of the meal may also be determined using an insulin model. Therefore, the amount of residual glucose (the amount of glucose remaining after subtracting the total amount of glucose level decrease due to active insulin from the total amount of glucose level increase due to the meal) may be determined. The amount of residual glucose may be converted into an amount of residual carbohydrates. The amount of residual glucose or residual carbohydrates may be used to determine the dose of a correction bolus (also referred to as the remedial dose or supplemental dose) for the patient, before the patient's blood glucose level may otherwise reach a high level or a postprandial settling value, such that the correction bolus may be delivered to the patient early to keep the blood glucose level within the desired range. For example, for a more aggressive correction bolus, the dose of the correction bolus may be determined based on both the residual carbohydrates (RC) and the current BG level *(BG)* at a given time according to:

$$B = \frac{RC}{ICR} + \frac{(BG - Target)}{ISF} - IOB. \qquad (3)$$

As a result, the number of correction boluses used to counteract a meal may be reduced, and the clinical outcomes (e.g., time in range) may be improved.

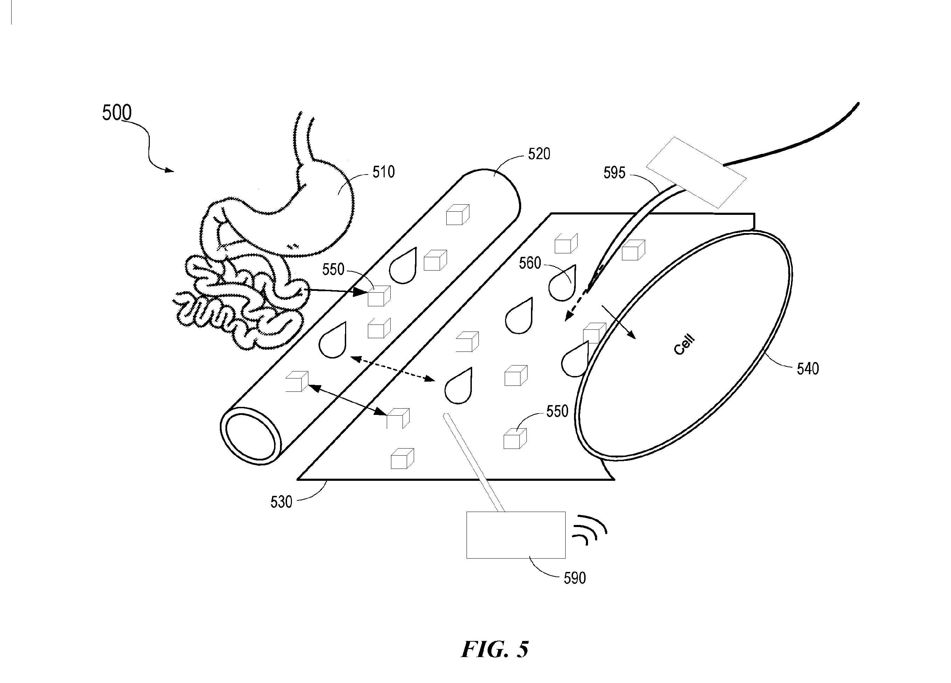

FIG. 5 illustrates a model 500 showing examples of glucose conversion, transportation, consumption, and measurement in a human body, and insulin delivery, transportation, and consumption in the human body for glucose metabolism according to certain embodiments. Based on model 500, a physiological model for simulating the glycemic response of a person may be determined. As illustrated, the digestive tract 510 (gut) of the human body may digest the food and convert food into glucose 550, which may be absorbed into blood stream 520, for example, at the small intestine and/or the large intestine. Thus, the glucose in digestive tract 510 may be increased due to food intake and may be reduced due to the absorption into blood stream 520.

Glucose 550 may be transported to other parts of the human body by blood stream 520, and may enter the interstitial fluid 530 between cells 540 at capillaries of the body. Some glucose 550 in interstitial fluid 530 may enter cells 540 with the facilitation of insulin 560 and used by cells 540. As described above, the liver of the human body may absorb excessive glucose and convert it into glycogen for storage, and may release the stored form of glucose into blood stream 520 to help increase the glucose level when the glucose level is low. Thus, the glucose concentration in blood stream 520 may be increased by glucose absorbed from meal and glucose generated by the liver, and may be reduced due to glucose entering interstitial fluid 530 and consumption by cells 540 as facilitated by insulin 560. The glucose in interstitial fluid 530 may be increased by glucose from blood stream 520 and may be reduced due to consumption by cells 540 as facilitated by insulin 560. The glucose concentration in interstitial fluid 530 may be measured by a glucose sensor 590. The glucose concentration in blood stream 520 may be estimated from the measured glucose concentration in interstitial fluid 530.

Insulin 560 may be secreted by the pancreas and enter blood stream 520. Blood stream 520 may carry insulin 560 to other parts of the human body, such that insulin 560 may enter interstitial fluid 530 to facilitate the utilization of glucose 550. For diabetic patient, at least some insulin may be injected into interstitial fluid 530 by an insulin infusion device 595, and may enter blood stream 520 and be carried to other parts of the human body. Thus, the concentration of insulin 560 in blood stream 520 may be reduced due to the transferring to interstitial fluid 530 and may be increased due to the transferring of delivered insulin (e.g., injected or infused to interstitial fluid 530 by insulin infusion device 595) from interstitial fluid 530. The concentration of insulin 560 in interstitial fluid 530 may be increased due to insulin delivered by insulin infusion device 595 and transported by blood stream 520, and may be reduced due to the consumption at cells 540.

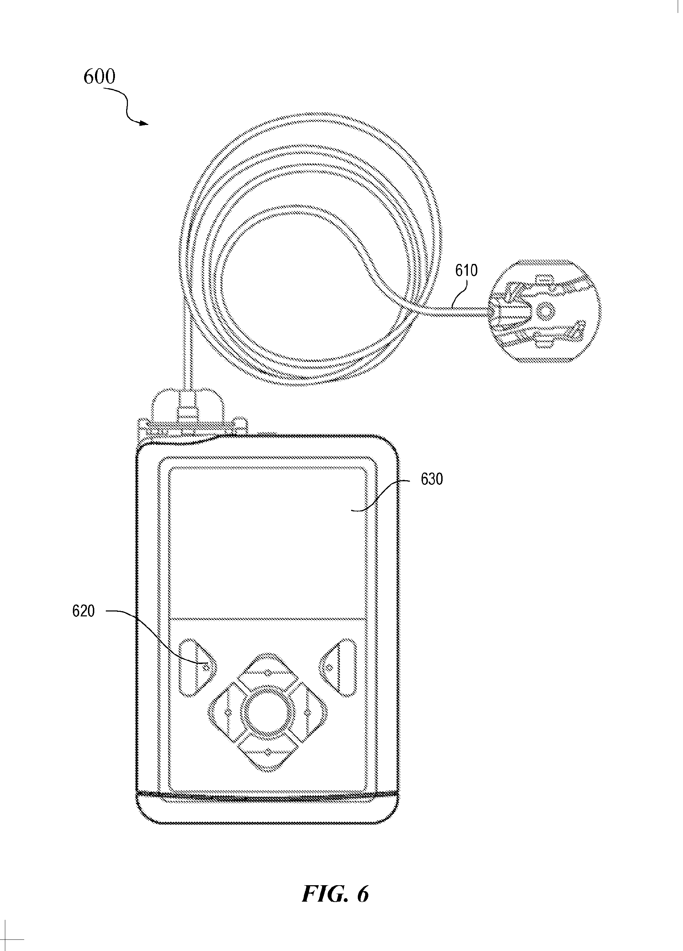

FIG. 6 depicts an example of a delivery device 600, which may implement a delivery device described above (e.g., delivery device 202, insulin delivery subsystem 330, or insulin infusion device 595). Delivery device 600 of FIG. 6 is merely provided by way of example, and other types of insulin delivery devices and other techniques different from those described above are contemplated to be within the scope of the present disclosure. In an insulin delivery device implemented using delivery device 600, insulin delivery may be performed based on internal communication between a central computing module (e.g., a microprocessor of delivery device 600) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., computing device 206 of FIG. 2) communicatively coupled to a remote or cloud computing system (e.g., remote/cloud computing system 208 of FIG. 2). Delivery device 600 may communicate various event data toward the remote or cloud computing system, which may communicate insulin delivery determinations toward delivery device 600, in accordance with the techniques described herein.

Delivery device 600 may provide insulin through a small tube 610 configured for fluidic connection with a cannula inserted subcutaneously. Delivery device 600 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. **In** the depicted example, delivery device 600 includes a user interface having button elements 620 that can be manipulated to administer a bolus of insulin, to change therapy settings, to change user preferences, to select display features, and the like. Delivery device 600 may also include a display device 630 that can be used to present various types of information or data to the user. **In**

accordance with aspects of the present disclosure, a user of delivery device 600 may use button elements 620 to input certain event data (e.g., event type, event start time, event details, etc.), and the user inputs can be confirmed using display device 630.

In some embodiments, delivery device 600 may be used to implement some techniques disclosed herein using, for example, the central computing module of delivery device 600 and/or a computing device (e.g., computing device 206 of FIG. 2 or remote/cloud computing system 208) communicatively coupled to delivery device 600. For example, delivery device 600 may be used to implement a physiological model for simulating the glycemic response of a patient, estimating parameters associated with the patient's physiological response (e.g., glycemic response) to a meal based on the model, predicting the total amount of glucose level increase that may result from the meal using the physiological model with the determined meal-specific parameters, calculating the amount of residual glucose and/or residual carbohydrates, determining the dose of a correction bolus for the patient, and/or predicting future glucose levels based on the model and insulin information.

Various physiological models can be used to simulate the glycemic response and metabolism of a human body. Some examples of the physiological model are described in, for example, Grosman et al., "Sensor-augmented pump-based customized mathematical model for Type 1 diabetes," DIABETES TECHNOLOGY & THERAPEUTICS, Volume 20, Number 3, pp. 207-221 (2018), and Grosman et al., "Fast-acting insulin aspart (Fiasp®) improves glycemic outcomes when used with MiniMed™ 670G hybrid closed-loop system in simulated trials compared to NovoLog®," Computer Methods and Programs in Biomedicine, 205 (2021) 106087, both of which are herein incorporated by reference in their entireties. Other physiological models can also be used in conjunction with the techniques described herein, including other types of models for different compartments of the physiological model. The physiological model may not be limited to mathematical models. For instance, a machine learning model could be trained to infer the glucose level and/or insulin level based on inputs similar to those used in the physiological model. In one implementation, the training of the machine learning model may involve using data associated with a general population as the initial training data set. Afterwards, the machine learning model may be further trained using data associated with the specific person.

At least some of the parameters in the physiological model (or parameters of a machine learning model) may be person-specific and/or meal-specific (e.g., parameters that determine conversion of ingested carbohydrates to glucose, absorption of insulin, etc.). In addition, some parameters may be subject to change over time (e.g., body weight, insulin sensitivity index, etc.). Therefore, while some parameters may be pre-stored or input by user, one or more model parameters may need to be determined based on the measured glycemic response to the specific meal at the specific time, such as by fitting at least some parts of the physiological model to the measurement data.

With some mathematical models, glucose levels can be simulated based on estimated physiological parameters. These physiological parameters may be estimated by fitting the mathematical models to observed trends of glucose level following recorded meal/insulin events. The set of physiological parameters that provide the closest fit to the observed glucose trend may be selected as the best estimated physiological parameters for the patient, and can then be used (with the mathematical models) to simulate the patient's glucose levels based on any imagined combination of meals and insulin doses.

In some embodiments, instead of using separate models for the meal response and insulin response, a single unified physiological model may be used to model both the glycemic response of a person to a meal and the metabolism of the person due to insulin. Therefore, the blood glucose level of the person as a function of time after a meal may be modeled using the unified physiological model and inputs to the physiological model, such as, for example, the amount of insulin delivered, the amount of food intake, and/or the measured SG values.

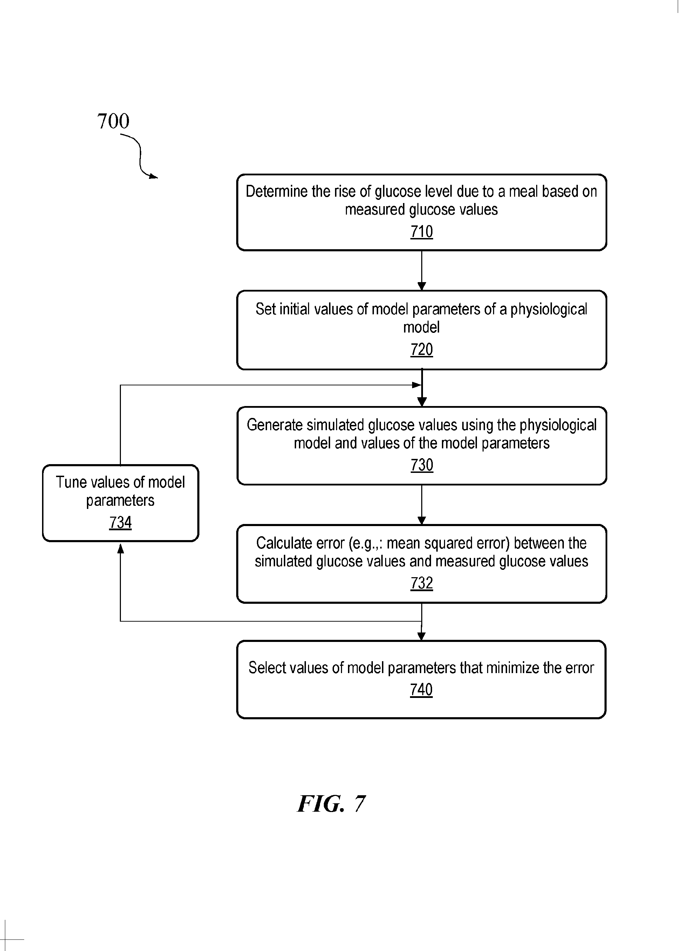

FIG. 7 includes a flowchart 700 illustrating an example of a method of determining appropriate values of parameters of a physiological model associated with a person and a meal for estimating blood glucose levels of the person and/or the dose of a correction bolus after the meal according to certain embodiments. The values of the parameters of the physiological model may be determined by fitting the glucose level or the rate of appearance of glucose in the blood due to the meal simulated using the physiological model to the estimated glucose level or rate of appearance of glucose in the blood due to the meal determined based on measured glucose data. Operations in flowchart 700 may be performed by, for example, computing device 206, monitoring device 204, delivery device 202, remote/cloud computing system 208, delivery device 600, or a combination thereof. Although flowchart 700 may describe the operations as a sequential process, some of the operations may be performed in parallel or concurrently. In addition, the order of the operations may be rearranged. The process may have additional steps not included in the figure. Some operations may be optional or may be omitted.

Operations in flowchart 700 may include, at block 710, determining a time series of the rise of glucose value due to a meal based on measured glucose values, for example, from a blood glucose monitor or a continuous glucose monitor. As described above, if no active insulin (e.g., meal bolus) is present, the rise of the glucose value due to the meal as a function of time may be approximately equal to the rise of glucose value as a function of time in the measured glucose values. In some cases, after a meal is detected, a meal bolus may be delivered, where the dose of the meal bolus may be determined using, for example, Equation 1. The rise of the glucose value due to the meal may then be determined as the sum of the measured rise of glucose value and the reduction of the glucose level caused by active insulin as described above. In some embodiments, a time series of the rate of appearance of glucose in the blood due to the meal may be determined based on the determined time series of the rise of glucose value due to the meal.

Operations at block 720 may include setting initial values of model parameters of a physiological model. The initial values may be, for example, typical values, average values, default values, previous values, or random values. At block 730, a time series of the rise of glucose value due to the meal at different time instants may be generated using the physiological model and the set values of the model parameters. In some embodiments, a time series of the rate of appearance of glucose in the blood due to the meal at different time instants may be generated using the physiological model and the set values of the model parameters. At block 732, the time series of rise of glucose value (or the rate of appearance of glucose in the blood) due to the meal generated using the physiological model may be compared with the time series of the rise of glucose value (or the rate of appearance of glucose in the blood) due to the meal determined based on the measured glucose values, and the error of the physiological model (e.g., a mean or accumulated squared error) may be determined based on the difference between the value estimated using the model and the value determined based on the measurement at each time instant of the time series. At block 734, the values of the model parameters may be tuned to reduce the error of the physiological model. Operations at blocks 730-734 may be performed iteratively to minimize the error of the physiological model. At block 740, the values of the model parameters of the physiological model that can minimize the error of the physiological model may be selected as the estimated values of the model parameters.

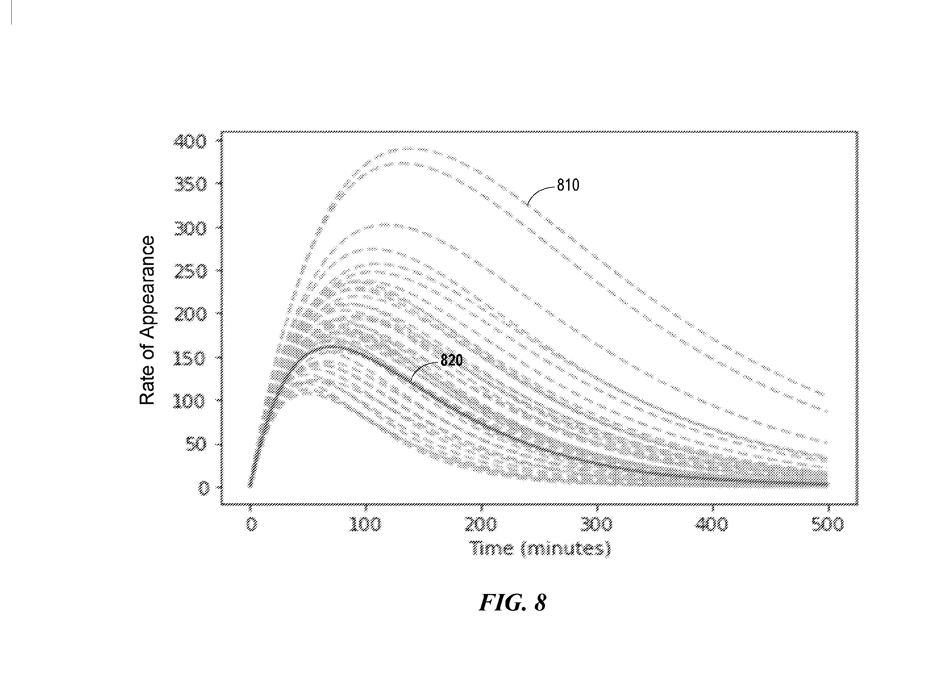

**FIG.** 8 illustrates an example of estimating values of parameters of a physiological model associated with a person and a meal according to certain embodiments. In the example shown in FIG. 8, curves 810 show a plurality of time series of the rate of appearance of glucose in the blood due to the meal estimated using the physiological model and a plurality of sets of values of the model parameters (e.g., meal parameters such as the carbohydrate to glucose conversion factor). The set of values of the model parameters of a curve 820 that has the smallest error with respect to the rate of appearance of glucose in the blood due to the meal determined using the measured glucose values as described above with respect to, for example, block 710, may be used as the estimated model parameters for the person and the meal.

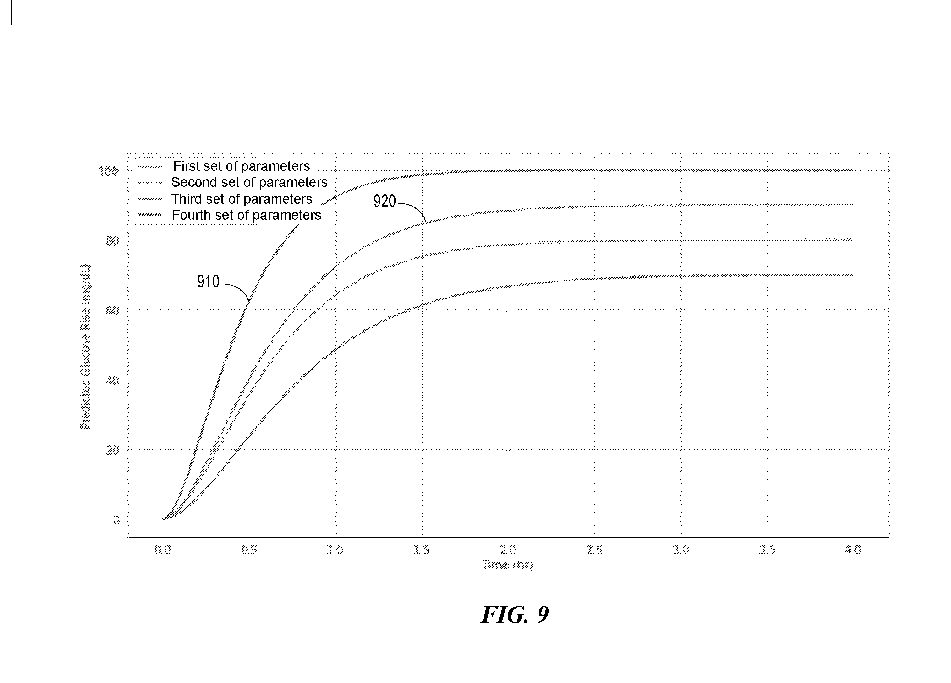

**FIG.** 9 illustrates another example of estimating values of parameters of a physiological model associated with a person and a meal according to certain embodiments. In the example shown in FIG. 9, curves 910 show a plurality of time series of the rise of blood glucose level due to the meal simulated using the physiological model and a plurality of sets of different values of the model parameters, such as meal parameters (e.g., carbohydrate to glucose conversion factor ). The set of values of the model parameters associated with a curve that has the smallest error with respect to the time series of the rise of the glucose value due to the meal determined using the measured glucose values may be used as the estimated values of the model parameters for the person and the meal. In the example shown in FIG. 9, a curve 920 represents such a curve, and the set of values of the model parameters associated with curve 920 may be used as the estimated values of the model parameters for the person and the meal.

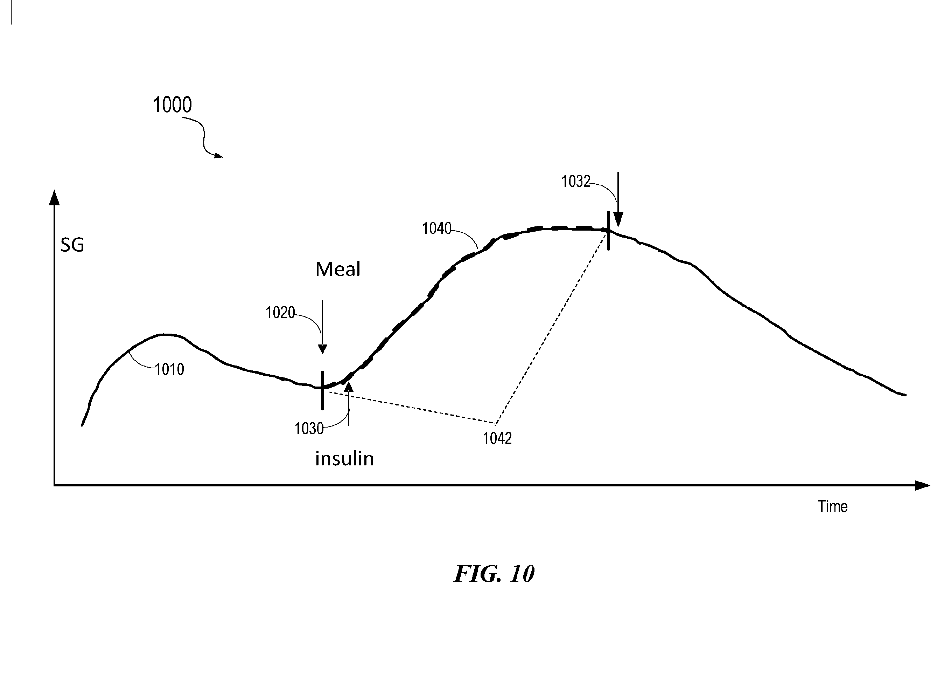

**FIG. 10** includes a diagram 1000 illustrating an example of using a fitted physiological model to estimate sensor glucose levels of a person after a meal and using the physiological model to determine an amount of residual carbohydrates (or residual glucose) and a correction bolus dose according to certain embodiments. **In** the example shown in FIG. 10, measured SG levels as a function of time are shown by a curve 1010. The SG levels may be measured using, for example, glucose sensor subsystem 310. As described above, small doses of basal bolus may be continuously delivered to the patient to maintain the blood glucose level of the patient within a certain range. When a meal 1020 is taken by the patient, if no additional insulin is delivered, the blood glucose level of the patient may increase quickly. To counter the glucose level increase caused by the meal, a meal bolus 1030 is delivered to the patient to prevent the blood glucose level from reaching a level that may cause hyperglycemia. The dose of meal bolus 1030 may be determined according to, for example, Equation 1. The postprandial SG values in a fitting time window 1042 may be measured and fitted to a physiological model that can be used to estimate the rise of glucose value $SG_{increase\ by\ meal}$ (or the rate of appearance of glucose in the blood) due to the meal and/or the reduction of glucose value $SG_{drop\ by\ insulin}$ due to active insulin (including meal bolus 1030) using techniques described above, for example, with respect to FIGS. 7-9. The observed SG values may be estimated using the fitted physiological model directly (e.g., when the physiological model is a unified model) or according to:

$$SG_{observed} = SG_{increase\ by\ meal} - SG_{drop\ by\ insulin}, \tag{4}$$

and may be shown by a curve 1040.

Based on the fitted physiological model, a new parameter - residual carbohydrate (RC) - may be determined and used in a bolus equation to determine the dose of a correction bolus 1032 for remedying the insufficiency of meal bolus 1030 or previous correction bolus(es). In one example, to determine the residual carbohydrate, residual glucose may be determined using the fitted physiological model with the estimated parameters that characterizes the person's glycemic response to the meal. For example, the overall rise of glucose level due to the meal may be estimated using a physiological model over a sufficiently long integration time period as shown by the peak value of curve 920 (or the total area under curve 820). The reduction of glucose level caused by active insulin may be estimated using the physiological model. The residual glucose (residual SG) that may not be counteracted by the active insulin may be determined according to:

$$\text{residual SG} = \text{glucose rise due to meal} - \text{glucose drop due to insulin}, \qquad (5)$$

where glucose rise due to meal may be a function of carbs, meal absorption, and the like, and glucose drop due to insulin may be a function of insulin sensitivity, insulin present in the blood, and the like. Based on the estimated residual SG, the residual carbohydrates RC may be determined according to:

$$RC = ICR \times \frac{Residual\,SG}{ISF}. \qquad (6)$$

In some implementations, the rise of glucose level due to a meal and/or the residual carbohydrates RC (or residual glucose) may be determined using a machine learning model, rather than the mathematical model described above.

Based on the determined residual carbohydrates (or residual glucose), a correction bolus may be calculated by an insulin calculator. For example, a more aggressive correction bolus may be determined based on both the *RC* and the current BG level at a given time and the target BG according to, for example, Equation 3. In some embodiments, a more conservative correction bolus may be determined based on the lower one of the doses determined based on, for example, *(1) RC/ICR (or residual SG/ISF)* and (2) Equation 2. In some embodiments, the correction bolus may be determined based on, for example, the higher one of the doses determined based on (1) *RC/ICR (or residual SG/ISF)* and (2) Equation 2. In some embodiments, the dose of the correction bolus may be a certain percentage of the correction bolus determined according to, for example, Equation 3. For example, a more aggressive correction bolus having a dose that is greater than about 50% (e.g., 75%) of the correction bolus determined according to Equation 3 may be used, or a more conservative correction bolus having a dose that is less than about 50% (e.g., 25%) of the correction bolus determined according to Equation 3 may be used.

In some embodiments, a meal bolus may not be delivered, and a correction bolus may be calculated and delivered using techniques disclosed herein, where the measured rise of glucose level may be close to the estimated rise of the glucose level due to the meal because of a low amount of active insulin. In some embodiments, more than one correction bolus may be calculated and delivered at different time instants after a meal, such as at 0.5 hours, 1 hour, and/or 2 hours after the meal. In some embodiments, a meal may not be logged, and the meal intake may be detected based on the measured SG values and/or fitting the physiological model to the measured SG values.

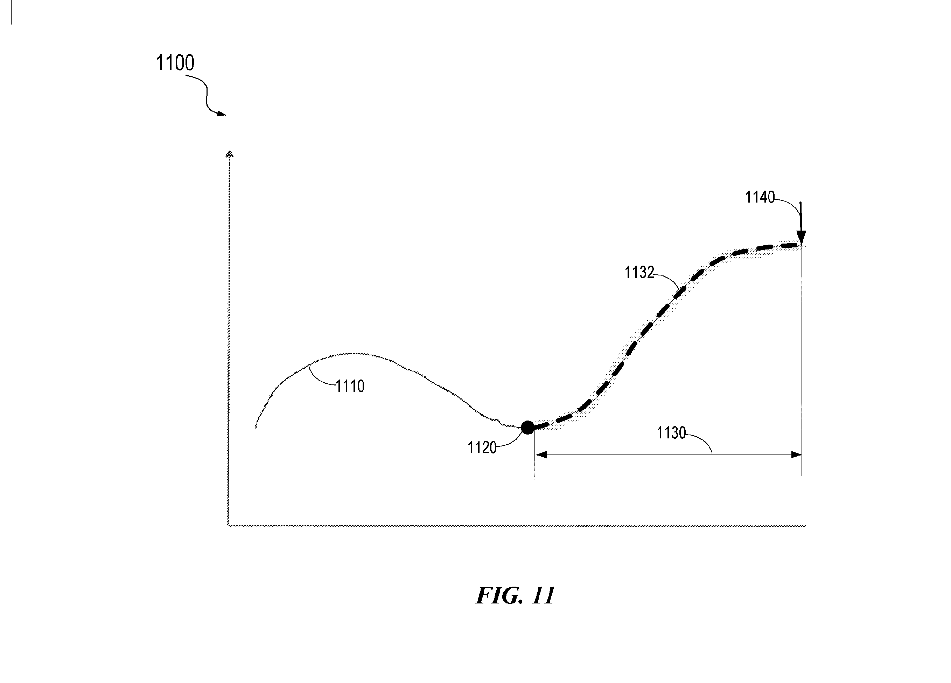

**FIG. 11** includes a diagram 1100 illustrating another example of using a fitted physiological model to estimate sensor glucose levels of a person after a meal and using the physiological model to determine an amount of residual carbohydrates and a correction bolus dosage according to certain embodiments. In the example shown in FIG. 11, measured SG levels as a function of time are shown by a curve 1110. The SG levels may be measured using, for example, glucose sensor subsystem 310. After a meal 1120 is taken by the patient, the SG values in a fitting time window 1130 may be measured and fitted to a physiological model that can be used to determine the rise of glucose value $SG_{increase\,by\,meal}$ (or the rate of appearance of glucose in the blood) due to the meal using techniques described above, for example, with respect to FIGS. 7-9. The observed SG values may be estimated using the fitted physiological model (e.g., Equation 4) and may be shown by a curve 1132, where the active insulin does not include a meal bolus at around the meal time in the illustrated example. The residual glucose or residual carbohydrates may be determined as described above (e.g., with respect to FIG. 10), and a late correction bolus 1140 may be calculated as described above with respect to FIG. 10 and delivered to the person.

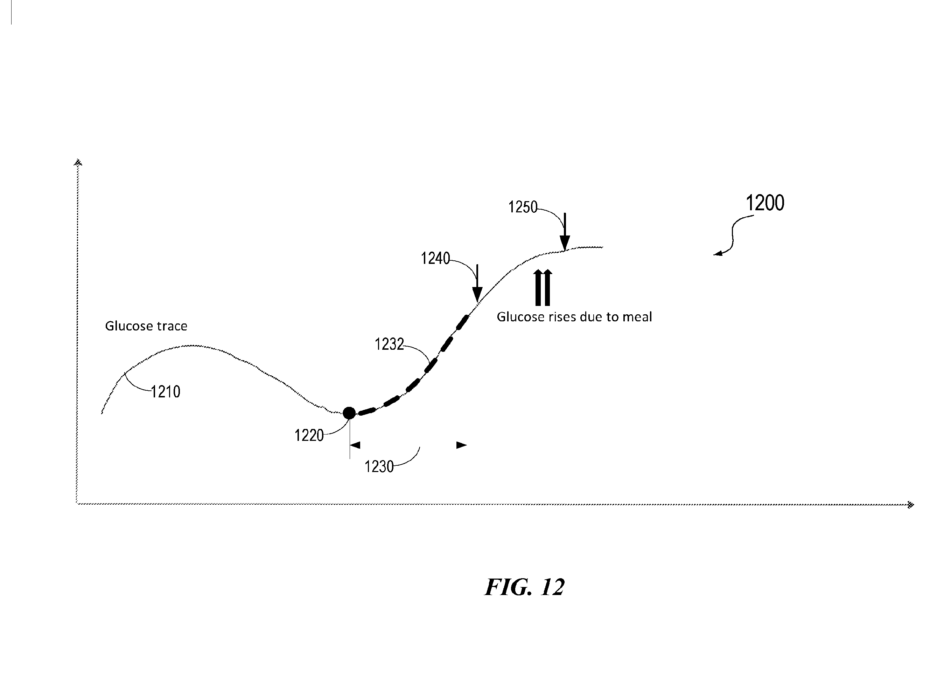

**FIG. 12** includes a diagram 1200 illustrating yet another example of using a fitted physiological model to estimate sensor glucose levels of a person after a meal and using the physiological model to determine an amount of residual carbohydrates and a correction bolus dosage according to certain embodiments. In the example shown in FIG. 12, measured SG levels as a function of time are shown by a curve 1210. The SG levels may be measured using, for example, glucose sensor subsystem 310. As described above, small dosages of basal bolus may be continuously delivered to the patient. After a meal 1220 is taken by the patient, the SG values in a fitting time window 1230 may be measured and fitted to a physiological model that can be used to estimate the rise of glucose value $SG_{increase\,by\,meal}$ (or the rate of appearance of glucose in the blood) due to the meal using techniques described above, for example, with respect to FIGS. 7-9. The observed SG values may be estimated using the fitted physiological model and/or Equation 4, and may be shown by a curve 1232, where the active insulin may not include a meal bolus in the illustrated example. The residual glucose or residual carbohydrates may be determined as described above, and a correction bolus 1240 may be calculated as described above with respect to FIG. 10 and delivered to the person.

After the delivery of correction bolus 1240, the SG levels may continue to be measured using, for example, glucose sensor subsystem 310, and may be fitted to the physiological model in another fitting time window to update the parameters of the model. Residual glucose or residual carbohydrates at a later time may be determined again using techniques disclosed herein, taking into consideration of the effect of correction bolus 1240. A second correction bolus 1250 may then be calculated as described above with respect to FIG. 10 and delivered to the person. In some embodiments, the physiological model fitted based on the measured glucose values in fitting time window 1230 may continue to be used to predict feature glucose value. In some embodiments, second correction bolus 1250 may be determined based on measured or predicted glucose level and Equation 2.

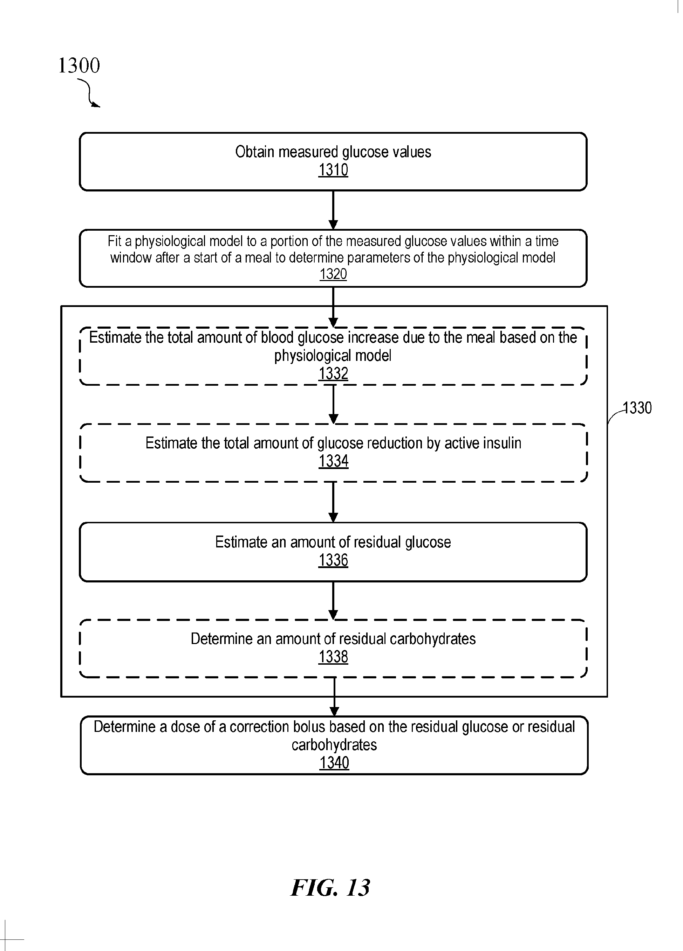

FIG. 13 includes a flowchart 1300 illustrating an example of a process of estimating parameters of a physiological model for a person and a meal and using the physiological model to determine an amount of residual carbohydrates and a correction bolus dosage according to certain embodiments. Operations in flowchart 1300 may be performed by, for example, computing device 206, monitoring device 204, delivery device 202, remote/cloud computing system 208, controller 320, delivery device 600, or a combination thereof. Although flowchart 1300 may describe the operations as a sequential process, some of the operations may be performed in parallel or concurrently. In addition, the order of the operations may be rearranged. The process may have additional steps not included in the figure. Some operations may be optional or may be omitted.

In the illustrated example, operations in block 1310 of flowchart 1300 may include obtaining measured glucose values of a patient. The glucose values may be blood glucose values measured using a blood glucose monitor or SG values (estimated blood glucose values) measured using a CGM sensor. In some embodiments, active insulin information and/or insulin delivery information may also be obtained. In some embodiments, a meal may be detected, for example, by a gesture sensor, based on changes in the measured SG levels, logged by a user, and the like. In one example, the start of a meal may be detected using a gesture sensor, or based on the measured glucose values of the person, for example, using a machine learning model.

Operations in block 1320 may include fitting a physiological model to a portion of the measured glucose values (e.g., SG values) within a time window after the start of a meal to determine values of parameters of the physiological model that characterizes the person's glycemic response to the specific meal. As described above, the physiological model may be a mathematical model or a machine learning model for glycemic response prediction. The physiological model may include a group of separate models (e.g., equations) for different compartments, or may include a single unified model. In one example, the physiological model may be a mathematical model as described above with respect to FIG. 6. The parameters of the physiological model to be determined for the patient and the specific meal may indicate, for example, a rate of glucose absorption and a carbohydrate-to-glucose conversion factor (or a product of carbohydrate-to-glucose conversion factor and the amount of carbohydrates in the meal).

Examples of fitting the physiological model to the measured glucose values to estimate the parameters of the physiological model are described above with respect to, for example, FIGS. 7-9. In one example, a time series (e.g., a curve) of estimated rise of blood glucose level (or the rate of appearance of glucose in the blood) due to the meal may be determined based on the measured glucose values. A plurality of time series (e.g., curves) of simulated rise of blood glucose level (or the rate of appearance of glucose in the blood) due to the meal may be generated using the physiological model and a plurality of sets of values of the parameters of the physiological model. A time series of simulated rise of blood glucose level due to the meal that best matches the time series of estimated rise of blood glucose level due to the meal may be selected from the plurality of time series of simulated rise of blood glucose level due to the meal. The selected time series of simulated rise of blood glucose level due to the meal may have the lowest error (e.g., mean squared error) from the time series of estimated rise of blood glucose level due to the meal among the plurality of time series of simulated rise of blood glucose level due to the meal. The parameters of the physiological model may be set to a set of values of the parameters of the physiological model used to generate the selected time series of simulated rise of blood glucose level due to the meal.

In some embodiments, determining the time series of estimated rise of blood glucose level due to the meal may include estimating a time series of reduction of blood glucose level due to active insulin, and determining the time series of estimated rise of blood glucose level due to the meal by adding the time series of reduction of blood glucose level due to active insulin to the portion of the measured glucose values. The active insulin may include basal insulin, a meal bolus, or a combination thereof. In some embodiments, fitting the physiological model to the portion of the measured glucose values may be based on a matching between an estimated rate of appearance of glucose in blood determined based on the measured glucose values, and a simulated rate of appearance of glucose in blood determined using the physiological model.

Operations in block 1330 may include estimating, using the physiological model and the determined parameters of the physiological model, an amount of residual glucose or residual carbohydrate of the meal that may not be counteracted by insulin already delivered to the person (e.g., active insulin). In one example, estimating the amount of residual glucose or residual carbohydrate of the meal may include estimating the total amount of blood glucose increase due to the meal based on the physiological model and the determined parameters of the physiological model at block 1332; estimating the total amount of glucose reduction by active insulin using an insulin model (e.g., based on an insulin sensitivity factor and an amount of active insulin) at block 1334; determining at block 1336 the amount of residual glucose as a difference between the total amount of blood glucose increase due to the meal estimated at block 1332 and the total amount of glucose reduction by active insulin determined at block 1334; and optionally determining at block 1338 the residual carbohydrates based on residual glucose determined at block 1336. In one example, the total amount of blood glucose increase due to the meal may be determined based on the area under a curve of the rate of appearance of blood glucose estimated using the fitted physiological model. The total amount of glucose reduction by active insulin may be determined, for example, based on the area under the curve of the active insulin in the plasma estimated using the active insulin level in the blood and the patient's insulin sensitivity (SI). The residual glucose may be determined, for example, using Equation 5. If the residual glucose is greater than zero, the residual carbohydrates in the patient's body may be determined based on the residual glucoses. In another example, operations in blocks 1332-1336 may be performed in one operation using a unified physiological model.

Operations in block 1340 may include determining the dose of a correction bolus based on the residual glucose or residual carbohydrates. In one example, determining the dose of the correction bolus may include calculating (1) a first insulin dose based on the residual carbohydrates (e.g., RC/ICR) or residual glucose (e.g., residual SG/ISF) and (2) a second insulin dose based on the current glucose level and target glucose level according to Equation 2, and determining the dose of the correction bolus based on the first insulin dose and the second insulin dose. For example, in various embodiments, the lower one of the first insulin dose and the second insulin dose may be used for the correction bolus (for a more conservative correction bolus), the sum of the first insulin dose and the second insulin dose may be used for the correction bolus (for a more aggressive correction bolus), a certain percentage of the sum of the first insulin dose and the second insulin dose may be used for the correction bolus, the higher one of the first insulin dose and the second insulin dose may be used for the correction bolus, and the like.

In some embodiments, operations in flowchart 1300 may be performed again at a later time after the meal to determine an additional correction bolus. In some embodiments, based on the physiological model with the determined parameters for the person and the meal and the correction bolus administered, future glucose levels of the person may be predicted. In some embodiments, the physiological model with the determined parameters for the person and the meal may be used to determine the appropriate insulin delivery dose/timing to keep the patient's future glucose levels within a desired range. For example, the insulin calculator may calculate a correction insulin dose (and determine the delivery time), and the physiological model may be used to predict future glucose levels based on the calculated correction insulin dose. The correction insulin dose (and/or the delivery time) may be adjusted based on the prediction (e.g., whether the glucose levels may be out of range). The final correction insulin dose (and the delivery time) that may achieve the desired future glucose levels based on the prediction using the physiological model may be administered to the patient as the correction bolus.

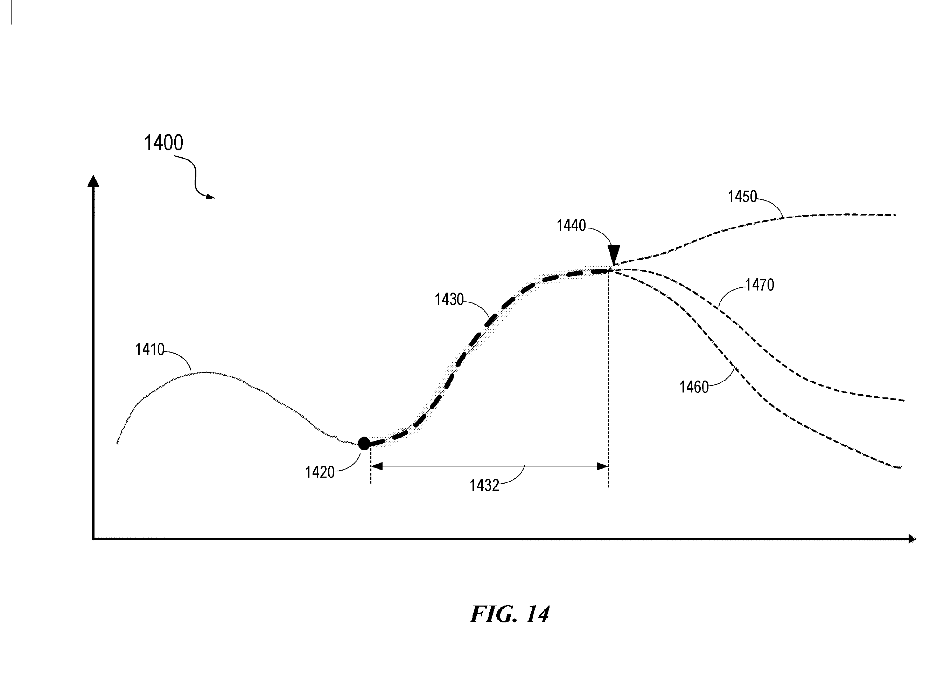

FIG. 14 includes a diagram 1400 illustrating an example of estimating parameters of a physiological model for a person and a meal and using the physiological model to predict future glucose levels according to certain embodiments. In the example shown in FIG. 14, measured SG levels as a function of time are shown by a curve 1410. The SG levels may be measured using, for example, glucose sensor subsystem 310. As described above, small dosages of basal bolus may be continuously delivered to the patient to try to maintain the blood glucose level of the patient to within a certain range. When a meal 1420 is taken by the patient, if no additional insulin is delivered, the blood glucose level of the patient may increase quickly. In some embodiments, to counter the glucose level increase caused by the meal, a meal bolus may be delivered to the patient to prevent the blood glucose level from reaching a level that may cause hyperglycemia. The dose of the meal bolus may be determined according to, for example, Equation 1. In some circumstances, the user may not log the meal or the meal may not be detected, and thus a meal bolus may not be delivered at a time around the start of the meal. In the example shown in FIG. 14, a meal bolus may or may not be delivered to the patient.

The SG values of the person within a fitting time window 1432 may be measured and fitted to a physiological model that can be used to determine the glycemic response of the person to the meal, such as the rise of glucose level due to the meal or the rate of appearance of glucose in the blood due to the meal, using techniques described above, for example, with respect to FIGS. 7-9. A curve 1430 in FIG. 14 shows the estimated SG values determined using the fitted physiological model, where curve 1430 may match the rise of glucose value due to the meal estimated based on the measured glucose value within fitting time window 1432.

As described above, in embodiments where active insulin is present in the body (e.g., a meal bolus is delivered at around the time of the meal), the reduction of glucose value due to active insulin (including the meal bolus) may be calculated and added to the measured glucose values to estimate the rise of the glucose value due to the meal as described above. The sum of the amount of the reduction of glucose value due to active insulin and measured SG value as a function of time may be fitted to the physiological model that estimates the rise of glucose level due to the meal, thereby determining the parameters of the model for the person and the meal, as described above with respect to, for example, FIGS. 10-13. In embodiments where the physiological model may be a unified model that can model both the glycemic response of a person to a meal and the metabolic actions of the person due to insulin, the measured SG values and insulin delivered may be used directly to fit the physiological model to determine unknown parameters of the physiological model.

Based on the fitted physiological model, the future rise of the glucose level of the person due to the meal may be predicted. The glucose level of the person may be predicted, for example, 1-4 hours into the future. One example of the predicted future rise of the glucose level of the person due to the meal only (without considering active insulin) may be shown by a curve 1450. Due to active insulin present in the body of the person, the glucose level of the person may reduce as shown by a curve 1460, if no meal impact (e.g., no residual carbohydrates) is considered. The amount of reduction of the glucose level due to the active insulin may be predicted, for example, according to the physiological model. The future blood glucose level of the person may be predicted based on the difference between the future rise of the glucose level of the person due to the meal and the reduction of the glucose level of the person due to the active insulin. A curve 1470 in FIG. 14 shows an example of the predicted future blood glucose level of the person as a function of time, when both the effect of the residual carbohydrates and the effect of active insulin are taken into consideration.

In some embodiments, the physiological model with the determined parameters for the person and the meal may be used to determine the appropriate dose (and/or time) of the correction bolus to keep the patient's future glucose levels within the desired range. For example, the insulin calculator may calculate a correction insulin dose (and determine the delivery time) as described above with respect to FIG. 13. In some implementations, the correction insulin dose may be set to a conservative value, a default value, a random value within a range, and the like. The physiological model may be used to predict the future glucose levels based on the calculated or selected correction insulin dose as described above. The correction insulin dose (and/or the delivery time) may be adjusted based on the results of the future glucose level prediction (e.g., whether the glucose levels may be out of range). The final correction insulin dose (and the delivery time) that may achieve the desired future glucose levels based on the prediction using the physiological model may be administered to the patient.

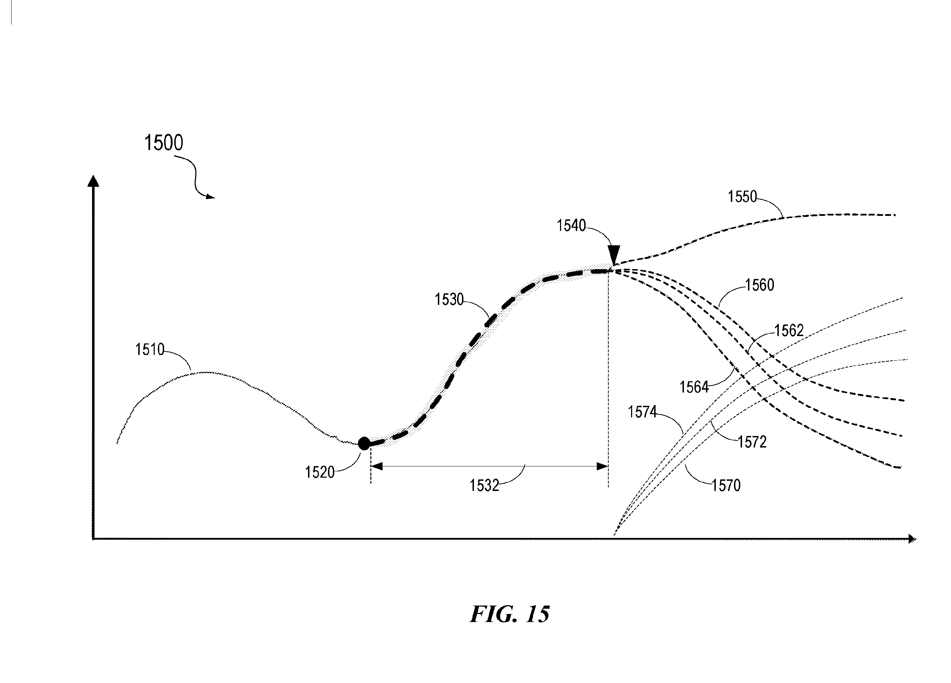

**FIG. 15** includes a diagram 1500 illustrating an example of estimating parameters of a physiological model for a person and a meal and using the physiological model to predict future glucose levels and/or determine a correction bolus dosage according to certain embodiments. In the example shown in FIG. 15, measured SG levels as a function of time are shown by a curve 1510. The SG levels may be measured using, for example, glucose sensor subsystem 310. As described above, small dosages of basal bolus may be continuously delivered to the patient to try to maintain the blood glucose level of the patient to within a certain range. When a meal 1520 is taken by the patient, if no additional insulin is delivered, the blood glucose level of the patient may increase quickly. In some embodiments, to counter the glucose level increase caused by the meal, a meal bolus may be delivered to the patient to prevent the blood glucose level from reaching a level that may cause hyperglycemia. The dose of the meal bolus may be determined according to, for example, Equation 1. In some circumstances, the user may not log the meal or the meal may not be detected, and thus a meal bolus may not be delivered at a time around the start of the meal. In the example shown in FIG. 15, a meal bolus may or may not be delivered to the person.

The SG values of the person within a fitting time window 1532 may be measured and fitted to a physiological model that can be used to determine the glycemic response of the person to the meal, such as the rise of glucose level due to the meal or the rate of appearance of glucose in the blood due to the meal, using techniques described above, for example, with respect to FIGS. 7-9. A curve 1530 in FIG. 15 shows the estimated SG values determined using the fitted physiological model, where curve 1530 may match the rise of glucose value due to the meal estimated based on the measured glucose value within fitting time window 1532. As described above, in embodiments where active insulin is present in the body (e.g., a meal bolus is delivered at around the time of the meal), the reduction of glucose value due to the active insulin may be determined and may be added to the measured glucose values to fit the model and estimate the rise of the glucose value due to the meal as described above. In embodiments where the physiological model models both the glycemic response of a person to a meal and the metabolic actions of the person due to insulin, the measured SG values and insulin delivered may be used directly to fit the physiological model to determine unknown parameters of the physiological model.

Based on the fitted physiological model, the future rise of the glucose level of the person due to the meal may be predicted. One example of the predicted future rise of the glucose level of the person due to the meal only (without considering active insulin) may be shown by a curve 1550. Based on active insulin present in the body of the person, the reduction of the glucose level of the person due to the active insulin may be predicted, for example, according to the physiological model. As such, the future blood glucose level of the person as a function of time may be predicted based on the difference between the future rise of the glucose level of the person due to the meal and the reduction of the glucose level of the person due to the active insulin.

As described above, in some embodiments, the total rise of glucose level due to the meal may be determined using the physiological model, and the total reduction of glucose value due to active insulin (if any) may be determined based on the active insulin using an insulin model or the physiological model. The residual glucose or residual carbohydrate that may not be counteracted by the active insulin may then be determined based on the difference between the total rise of glucose level due to the meal and the total reduction of glucose value due to the active insulin (if any). The dose of a correction bolus 1540 may then be calculated as described above with respect to, for example, FIGS. 10-13, and correction bolus 1540 may be delivered to the person accordingly.

In some embodiments, the future glucose level of the person may be predicted based on the physiological model with the determined parameters for the person and the meal and correction bolus 1540 administered. For example, the future glucose level of the person may be the difference between the predicted future rise of the glucose level of the person due to the meal as shown by curve 1550 and the reduction of glucose level due to the active insulin including correction bolus 1540. In one example, the reduction of glucose level due to the active insulin including correction bolus 1540 after the delivery of correction bolus 1540 may be shown by a curve 1570, and the predicted future glucose level of the person after the delivery of correction bolus 1540 may be shown by a curve 1560, which may be determined based on the difference between curve 1550 and curve 1570.

In some embodiments, a single unified physiological model may model both the glycemic response of a person to a meal and the metabolic actions of the person due to insulin, and thus the future blood glucose level of the person as a function of time may be predicted using the fitted physiological model and inputs to the physiological model, such as the amount of insulin delivered, the amount of food intake, and/or the measured SG values.

In some embodiments, the physiological model with the determined parameters for the person and the meal may be used to determine the appropriate dose (and/or time) of the correction bolus to keep the patient's future glucose levels within the desired range. For example, the insulin calculator may calculate a correction insulin dose (and determine the delivery time) as described above with respect to FIG. 13. In some implementations, the correction insulin dose may be set to a conservative value, a default value, a random value within a range, and the like. The physiological model may be used to predict the future glucose levels based on the calculated or selected correction insulin dose as described above with respect to FIG. 14. The correction insulin dose (and/or the delivery time) may then be adjusted based on the results of the future glucose level prediction (e.g., whether the glucose levels may be out of range). The final correction insulin dose (and the delivery time) that may achieve the desired future glucose levels based on the prediction using the physiological model may be administered to the patient.

For example, as shown in FIG. 15, the estimated reduction of glucose level due to the active insulin that includes a first dose of correction bolus may be shown by curve 1570, and the predicted future glucose levels of the person after the delivery of the first dose of correction bolus may be shown by curve 1560. The estimated reduction of glucose level due to the active insulin that includes a second dose of correction bolus may be shown by a curve 1572, and the predicted future glucose levels of the person after the delivery of the second dose of correction bolus may be shown by curve 1562. The estimated reduction of glucose level due to the active insulin that includes a third dose of correction bolus may be shown by a curve 1574, and the predicted future glucose levels of the person after the delivery of the third dose of correction bolus may be shown by curve 1564. The predicted future glucose levels of the person after the delivery of other doses of correction bolus may also be determined. The dose of the correction bolus (and the delivery time) that may achieve the desired future glucose levels based on the prediction using the physiological model may be selected, and the selected correction bolus may be administered to the person accordingly.

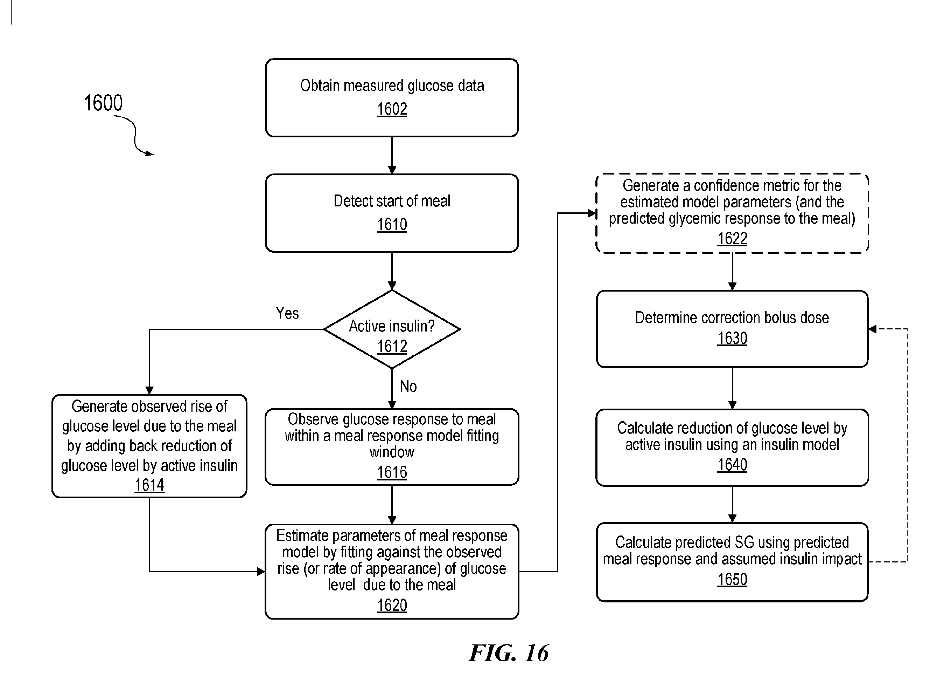

**FIG. 16** includes a flowchart 1600 illustrating an example of a process of estimating parameters of a physiological model for a person and a meal and using the physiological model to predict future glucose levels and/or determine a correction bolus dosage according to certain embodiments. Operations in flowchart 1600 may be performed by, for example, computing device 206, monitoring device 204, delivery device 202, remote/cloud computing system 208, delivery device 600, or a combination thereof. Although flowchart 1600 may describe the operations as a sequential process, some of the operations may be performed in parallel or concurrently. In addition, the order of the operations may be rearranged. The process may have additional steps not included in the figure. Some operations may be optional or may be omitted.

Operations of flowchart 1600 may include obtaining measured glucose data (e.g., SG values measured using a CGM sensor) at block 1602. At block 1610, the start of a meal may be logged by a user, detected based on the measured glucose data, or detected using other techniques, such as motion/gesture detectors (e.g., a camera or a motion sensor). If it is determined at block 1612 that active insulin is present (e.g., a meal bolus is delivered before, at, or after the start of the meal) during a fitting time window, the observed rise of glucose level due to the meal may be determined at block 1614 based on the measured rise of glucose level and the reduction of glucose level by the active insulin during the fitting time window. As described above, the reduction of glucose level by the active insulin may be determined using an insulin model (e.g., the product of insulin used and an insulin sensitivity factor) or a unified physiological or machine learning model. If it is determined at block 1612 that no active insulin is present within the fitting time window, the observed rise of glucose level due to the meal may be determined at block 1616 to be the measured rise of glucose level.

At block 1620, parameters of a physiological model (or a part of the physiological model) that describes the glycemic response of the person to the meal may be determined by fitting the physiological model to the observed rise of glucose level (or rate of appearance of glucose in the blood stream) due to the meal as described above with respect to, for example, FIGS. 7-15. Optionally, at block 1622, a confidence metric for the estimated model parameters (and the predicted glycemic response to the meal) may be determined, for example, based on the error between the observed rise of glucose level due to the meal and the predicted rise of glucose level due to the meal determined using the physiological model and the estimated model parameters.

At block 1630, the dose of a correction bolus may be determined using the physiological model and the estimated parameters of the physiological model for the person and the meal as described above. In some implementations, the dose of the correction bolus may be set to a conservative value, a default value, a random value within a range, and the like. In some embodiments, the dose of the correction bolus may be the minimum bolus needed to reduce the person's blood glucose level to a target glucose level or another appropriate level, for example, using Equation 2 or 3. In one example, the dose of the correction bolus may be determined based on the residual glucose or residual carbohydrates that may be determined based on the total rise of glucose level due to the meal determined using the physiological model and the reduction of glucose level due to active insulin determined using the physiological model as described above with respect to, for example, FIGS. 13-15.

At block 1640, the reduction of glucose level due to active insulin (e.g., including the correction bolus determined at block 1630) as a function of time may be predicted using an insulin model. At block 1650, the future blood glucose level of the person as a function of time may be predicted based on the difference between the predicted rise of glucose level due to the meal determined using the physiological model and the predicted reduction of glucose level due to the active insulin determined using the insulin model. As described above, in some embodiments, a unified physiological model or machine learning model may be able to model the reduction of glucose level due to active insulin, the increase of glucose level due to meal, and other responses and metabolism of a human body. Thus, operations at block 1640 and 1650 may be merged and can be performed by a unified physiological model that can predict the future blood glucose level of the person as a function of time based on inputs to the physiological model, such as, for example, the amount of insulin delivered, the amount of food intake, and/or the measured SG values.

In some embodiments, based on the predicted future blood glucose level of the person as a function of time, the dose of the correction bolus may be adjusted, and operations in block 1640 and 1650 may be performed again to determine new predicted future blood glucose level of the person as a function of time. This process may be iteratively performed to determine a dose of the correction bolus that may be used to achieve the desired predicted future blood glucose level of the person as a function of time.

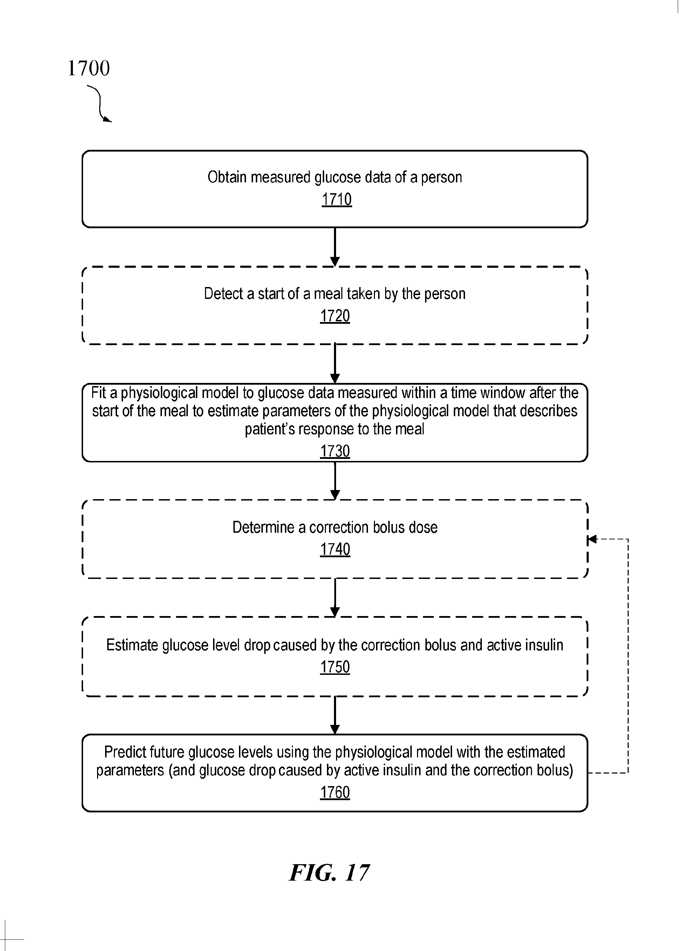

**FIG. 17** includes a flowchart 1700 illustrating an example of a process of estimating parameters of a physiological model for a person and a meal and using the physiological model to predict future glucose levels and/or determine a correction bolus dosage according to certain embodiments. Operations in flowchart 1700 may be performed by, for example, computing device 206, monitoring device 204, delivery device 202, remote/cloud computing system 208, delivery device 600, or a combination thereof. Although flowchart 1700 may describe the operations as a sequential process, some of the operations may be performed in parallel or concurrently. In addition, the order of the operations may be rearranged. The process may have additional steps not included in the figure. Some operations may be optional or may be omitted.

Operations at block 1710 may include obtaining measured glucose data of a person. The glucose data may be blood glucose levels measured using a blood glucose monitor or SG values (estimated blood glucose levels) measured using a CGM sensor. In some embodiments, active insulin information and/or insulin delivery information may also be obtained.

Optional operations at block 1720 may include detecting a start of a meal taken by the person. In some embodiments, the meal may be detected, for example, by a gesture sensor, based on changes in the measured SG levels, logged by a user, and the like. In one example, the start of the meal may be detected using a gesture sensor, or based on the measured glucose values of the person, for example, using a machine learning model.

Operations at block 1730 may include fitting a physiological model to a portion of the measured glucose values within a time window after a start of a meal to determine parameters of the physiological model that characterizes the person's glycemic response to the meal. As described above, the physiological model may be a mathematical model or a machine learning model for glycemic response prediction. The physiological model may include a group of separate models (e.g., equations) for different compartments (e.g., meal and insulin compartments), or may include a single unified model. In one example, the physiological model may be a mathematical model. The parameters of the physiological model to be determined for the patient and the specific meal may indicate, for example, a rate of glucose absorption and a carbohydrate-to-glucose conversion factor described above.

Examples of fitting the physiological model to the measured glucose values to estimate the parameters of the physiological model are described above with respect to, for example, FIGS. 7-15. In one example, a time series (e.g., a curve) of estimated rise of blood glucose level (or the rate of appearance of glucose in the blood) due to the meal may be determined based on the measured glucose values. A plurality of time series (e.g., curves) of simulated rise of blood glucose level (or the rate of appearance of glucose in the blood) due to the meal may be generated using the physiological model and a plurality of sets of values of the parameters of the physiological model. A time series of simulated rise of blood glucose level due to the meal that best matches the time series of estimated rise of blood glucose level due to the meal may be selected from the plurality of time series of simulated rise of blood glucose level due to the meal. The selected time series of simulated rise of blood glucose level due to the meal may have the lowest error (e.g., mean squared error) from the time series of estimated rise of blood glucose level due to the meal among the plurality of time series of simulated rise of blood glucose level due to the meal. The parameters of the physiological model may be set to a set of values of the parameters of the physiological model used to generate the selected time series of simulated rise of blood glucose level due to the meal.

In some embodiments, determining the time series of estimated rise of blood glucose level due to the meal may include estimating a time series of reduction of blood glucose level due to active insulin, and determining the time series of estimated rise of blood glucose level due to the meal by adding the time series of reduction of blood glucose level due to active insulin to the portion of the measured glucose values. The active insulin may include basal insulin, a meal bolus, or a combination thereof. In some embodiments, fitting the physiological model to the portion of the measured glucose values may be based on a matching between an estimated rate of appearance of glucose in blood determined based on the measured glucose values, and a simulated rate of appearance of glucose in blood determined using the physiological model.

Optional operations at block 1740 may include determining a correction bolus dose. The correction bolus dose may be determined using the physiological model and the estimated parameters of the physiological model for the person and the meal as described above. In some implementations, the dose of the correction bolus may be set to a conservative value, a default value, an initial value, a random value within a range, and the like. In some embodiments, the dose of the correction bolus may be the minimum bolus needed to reduce the person's blood glucose level to a target glucose level or another appropriate level, for example, using Equation 2 or 3. In one example, the dose of the correction bolus may be determined based on the residual glucose or residual carbohydrates that may be determined based on the total rise of glucose level due to the meal determined using the physiological model and the reduction of glucose level due to active insulin as described above with respect to, for example, FIG. 13.

Optional operations at block 1750 may include estimating glucose level drop caused by active insulin and the correction bolus, using an insulin model. Operations at block 1760 may include determining predicted glucose levels using the physiological model (with the estimated parameters) and glucose level drop caused by active insulin that includes the correction bolus. In some embodiments, based on the predicted future blood glucose level of the person as a function of time, the dose of the correction bolus may be adjusted, and operations in blocks 1750 and 1760 may be performed again to determine the new predicted future blood glucose level of the person as a function of time. Operations in blocks 1740-1760 may be iteratively performed to determine a dose of the correction bolus that may be used to achieve the desired predicted future blood glucose level of the person as a function of time. As described above, in some embodiments, a unified physiological model or machine learning model may be able to model the reduction of glucose level due to active insulin, the increase of glucose level due to meal, and other responses and metabolism of a human body. Thus, operations at block 1750 and 1760 may be merged and can be performed by a unified physiological model that can predict the future blood glucose level of the person as a function of time based on inputs to the physiological model, such as, for example, the amount of insulin delivered, the amount of food intake, and/or the measured SG values.

Embodiments of the methods disclosed herein may be implemented by hardware, software, firmware, middleware, microcode, hardware description languages, or any combination thereof. For example, the operations in the flowcharts may be performed by a server, a personal computer, a mobile device (e.g., a smartphone or a tablet), a medical device (e.g., a glucose sensor or an insulin delivery device), or a combination thereof. When implemented in software, firmware, middleware, or microcode, the program code or code segments to perform the associated tasks may be stored in one or more computer-readable media such as a storage medium, and may be executed by one or more processors to perform the associated tasks. The computer-readable media may include transitory or non-transitory computer-readable media, such as RAM, ROM, EEPROM, flash memory, solid-state drive, hard drive, CD, DVD, or any other medium that can be used to store desired program code in the form of instructions or data structures and that can be accessed by a computer. The one or more processors may include general purpose microprocessors, application specific integrated circuits (ASICs), graphic processing units (GPUs), network processing units (NPUs), digital signal processors (DSPs), field programmable logic arrays (FPGAs), and the like.

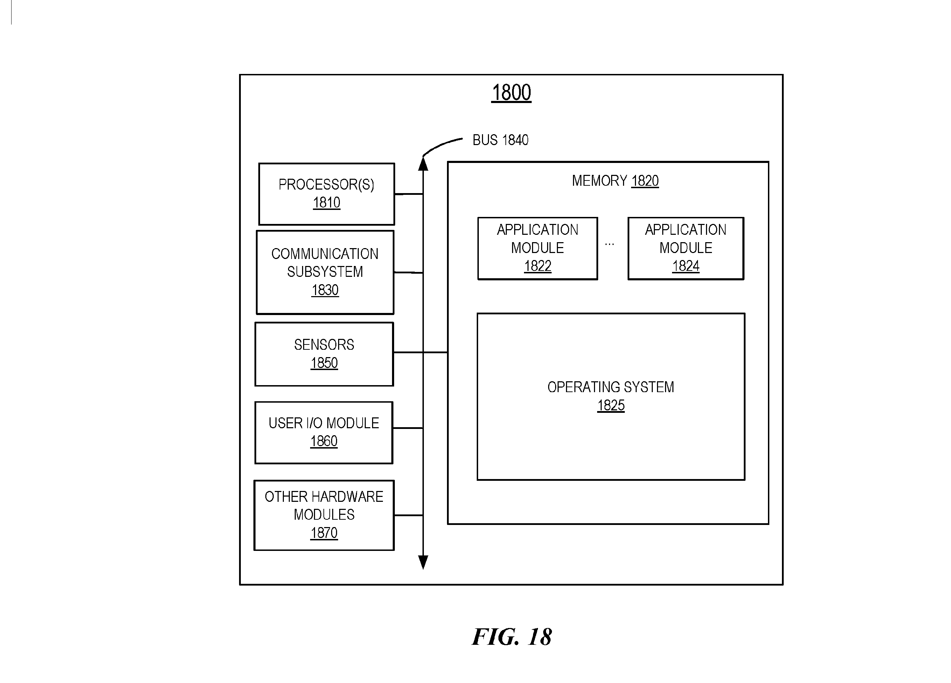

**FIG. 18** is a block diagram of an example of an electronic device 1800 that may implement some of the examples disclosed herein. For example, electronic device 1800 may be used to implement delivery device 202, monitoring device 204, computing device 206, and remote/cloud computing system 208, glucose sensor subsystem 310, controller 320, insulin delivery subsystem 330, glucose delivery subsystem 340, and glucagon delivery subsystem 350, or another device that may be used to calculate insulin dose and/or predict future blood glucose levels. In the illustrated example, electronic device 1800 may include one or more processor(s) 1810 and memory 1820. Processor(s) 1810 may be configured to execute instructions stored in memory 1820 for performing one or more of the methods described herein and other applications. Processor(s) 1810 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 1810 may be communicatively coupled with a plurality of components within electronic device 1800. To realize this communicative coupling, processor(s) 1810 may communicate with the other illustrated components across a bus 1840. Bus 1840 may be any subsystem adapted to transfer data within electronic device 1800. Bus 1840 may include a plurality of computer buses and additional circuitry to transfer data.

Memory 1820 may include one or more transitory and/or non-transitory storage devices, such as, for example, a static random access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 1820 may be distributed into different hardware modules.

Memory 1820 may include an operating system 1825 loaded therein. Operating system 1825 may be operable to initiate the execution of the instructions provided by application modules 1822-1824 and/or manage other hardware modules 1870, as well as interface with a communication subsystem 1830 which may include one or more wired and/or wireless transceivers. Operating system 1825 may be adapted to perform other operations across the components of electronic device 1800 including threading, virtualization, resource management, data storage control, and other similar functionality. In some embodiments, memory 1820 may store a plurality of application modules 1822 through 1824, which may include any number of applications. Examples of the applications may include an insulin calculator, a blood glucose level monitor or predictor, a glucose level management application, and the like. Application modules 1822-1424 may include particular instructions to be executed by processor(s) 1810. In some embodiments, certain applications or parts of application modules 1822-1424 may be executable by other hardware modules 1870.

Communication subsystem 1830 may include, for example, an infrared communication device, a wireless communication device and/or chipset (such as a Bluetooth® device, an IEEE 802.11 device, a Wi-Fi device, a WiMax device, cellular communication devices, etc.), and/or similar communication interfaces. One or more antennas (not shown) may be used for wireless communication as part of communication subsystem 1830 or as a separate component coupled to any portion of electronic device 1800, such as a wireless charging receiver or a near-field communication receiver. In some embodiments, communication subsystem 1830 may include circuits for wired communication technologies, such as Ethernet, coaxial communications, universal serial bus (USB), and the like. Communications subsystem 1830 may permit data to be exchanged with a network, other computer systems, and/or any other devices. For example, communications subsystem 1830 may be used to receive therapy determinations for therapeutic fluid (e.g., insulin) delivery, such as from a cloud computing system via an intermediary computing device (e.g., a controller) communicatively coupled to electronic device 1800, where processor(s) 1810 may, based on the therapy determinations, send commands to an actuator controller to cause the delivery of appropriate amounts of therapeutic fluid (e.g., insulin) to a user. In another example, communications subsystem 1830 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Sensor(s) 1850 may include, for example, a camera, an infrared sensor, an accelerometer, a pressure sensor, a temperature sensor, a proximity sensor, a magnetometer, a gyroscope, an inertial sensor (e.g., an inertial measurement unit (IMU)), an ambient light sensor, a position sensor, a depth sensor, a gesture detector, or any other similar module operable to provide sensory output and/or receive sensory input. In one example, sensor(s) 1850 may be used to implement a meal detector.

Input/output user interface 1860 may allow a user to send action requests to electronic device 1800 to perform particular actions, and may provide information (e.g., status of electronic device 1800, measurement results, alerts, etc.) to the user. Input/output user interface 1860 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 1810. In some embodiments, input/output user interface 1860 may include one or more output devices, such as a display, a speaker, a light emitting device, a haptic device, and the like, to provide feedback or alarm to the user.

In some embodiments, electronic device 1800 may include a plurality of other hardware modules 1870. Each of other hardware modules 1870 may be a physical module within electronic device 1800. While each of other hardware modules 1870 may be permanently configured as a structure, some of other hardware modules 1870 may be temporarily configured to perform specific functions or temporarily activated. Examples of other hardware modules 1870 may include, for example, an audio output and/or input module (*e.g.,* a microphone or speaker), a near field communication (NFC) module, a rechargeable battery, a battery management system, a wired/wireless battery charging system, an actuator controller, and the like. In some embodiments, one or more functions of other hardware modules 1870 may be implemented in software.

In one example, electronic device 1800 may be part of an insulin delivery device (e.g., a pump) that can deliver fast-acting insulin through a small tube configured for fluidic connection with a cannula inserted subcutaneously. Electronic device 1800 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like. The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in electronic device 1800. Similarly, functionality of one or more of the components can be distributed among the components in a manner different from the manner described above.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances.

The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure. While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. The aspects and features of the present disclosure and may be embodied in various forms. Thus, specific structural and functional details disclosed herein are not to be interpreted as limiting, but as a basis for the claims and as a representative basis for teaching one skilled in the art to variously employ the present disclosure in virtually any appropriately detailed structure.

With reference to the appended figures, components that can include memory can include non-transitory machine-readable media. The term "machine-readable medium," "processor-readable medium," or "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for interprocess communications, and different pairs of processes may use different techniques, or the same pair of processes may use different techniques at different times.

In view of this description embodiments may include different combinations of features. Implementation examples are described in the following numbered clauses:

Clause 1. A processor-implemented method comprising:

obtaining measured glucose values of a person;

fitting a physiological model to a portion of the measured glucose values within a time window after a start of a meal to determine meal-specific values of parameters of the physiological model that characterizes the person's glycemic response to the meal; and

predicting, using the physiological model and the meal-specific values of the parameters of the physiological model, a future blood glucose level of the person at a first time after the time window.

**Clause 2.** The processor-implemented method of Clause 1, wherein predicting the future blood glucose level of the person comprises:

estimating a reduction of glucose level by active insulin used up to the first time;

generating, using the physiological model and the meal-specific values of the parameters of the physiological model, a predicted rise of blood glucose level due to the meal at the first time; and

predicting the future blood glucose level of the person at the first time based on the reduction of glucose level by the active insulin used up to the first time and the predicted rise of blood glucose level due to the meal at the first time.

**Clause 3.** The processor-implemented method of Clause 2, wherein the active insulin includes a meal bolus, basal insulin, a correction bolus, or a combination thereof.

**Clause 4.** The processor-implemented method of any of Clauses 1-3, further comprising:

determining a dose of a correction bolus; and

causing delivery of the correction bolus to the person.

**Clause 5.** The processor-implemented method of any of Clauses 1-4, further comprising detecting the start of the meal based on the measured glucose values.

**Clause 6.** The processor-implemented method of any of Clauses 1-5, further comprising sending an alert or a notification to a user or an electronic device based on the predicted future blood glucose level of the person.

**Clause 7.** The processor-implemented method of any of Clauses 1-6, wherein the parameters of the physiological model characterize a carbohydrate-to-glucose conversion factor and a rate of glucose absorption.

**Clause 8.** The processor-implemented method of any of Clauses 1-7, wherein fitting the physiological model to the portion of the measured glucose values comprises:

determining, based on the measured glucose values, a time series of estimated rise of blood glucose level due to the meal;

generating, using the physiological model and a plurality of sets of values of the parameters of the physiological model, a plurality of time series of simulated rise of blood glucose level due to the meal; and

selecting, from the plurality of time series of simulated rise of blood glucose level due to the meal, a time series of simulated rise of blood glucose level due to the meal that best matches the time series of estimated rise of blood glucose level due to the meal,

wherein the meal-specific values of the parameters of the physiological model are set based on a set of values of the parameters of the physiological model used to generate the selected time series of simulated rise of blood glucose level due to the meal.

**Clause 9.** The processor-implemented method of Clause 8, wherein the selected time series of simulated rise of blood glucose level due to the meal has the lowest error with respect to the time series of estimated rise of blood glucose level due to the meal among the plurality of time series of simulated rise of blood glucose level due to the meal.

**Clause 10.** The processor-implemented method of Clause 8 or 9, wherein determining the time series of estimated rise of blood glucose level due to the meal comprises:

estimating a time series of reduction of blood glucose level due to active insulin; and

determining the time series of estimated rise of blood glucose level due to the meal by adding the time series of reduction of blood glucose level due to active insulin to the portion of the measured glucose values.

**Clause 11.** The processor-implemented method of any of Clauses 1-7, wherein fitting the physiological model to the portion of the measured glucose values is based on a matching between:

an estimated rate of appearance of glucose in blood determined based on the measured glucose values; and

a simulated rate of appearance of glucose in blood determined using the physiological model.

**Clause 12.** The processor-implemented method of any of Clauses 1-11, further comprising:

predicting, for each candidate dose of a plurality of candidate doses of a correction bolus, future blood glucose levels of the person using the physiological model and the meal-specific values of the parameters of the physiological model; and

selecting, from the plurality of candidate doses, a candidate dose as the dose of the correction bolus to be delivered to the person based on the predicted future blood glucose levels of the person for each candidate dose of the plurality of candidate doses of the correction bolus. **Clause 13.** A system comprising:

one or more processors; and

one or more processor-readable media storing instructions which, when executed by the one or more processors, cause performance of operations including:

obtaining measured glucose values of a person;

fitting a physiological model to a portion of the measured glucose values within a time window after a start of a meal to determine meal-specific values of parameters of the physiological model that characterizes the person's glycemic response to the meal; and

predicting, using the physiological model and the meal-specific values of the parameters of the physiological model, a future blood glucose level of the person at a first time after the time window.

**Clause 14.** The system of Clause 13, wherein predicting the future blood glucose level of the person comprises:

estimating a reduction of glucose level by active insulin used up to the first time;

generating, using the physiological model and the meal-specific values of the parameters of the physiological model, a predicted rise of blood glucose level due to the meal at the first time; and

predicting the future blood glucose level of the person at the first time based on the reduction of glucose level by the active insulin used up to the first time and the predicted rise of blood glucose level due to the meal at the first time.

**Clause 15.** The system of Clause 13 or 14, wherein:

the physiological model includes a mathematical model or a machine-learning model for glycemic response prediction; and

the parameters of the physiological model characterize a carbohydrate-to-glucose conversion factor and a rate of glucose absorption.

**Clause 16.** The system of any of Clauses 13-15, wherein fitting the physiological model to the portion of the measured glucose values comprises:

determining, based on the measured glucose values, a time series of estimated rise of blood glucose level due to the meal;

generating, using the physiological model and a plurality of sets of values of the parameters of the physiological model, a plurality of time series of simulated rise of blood glucose level due to the meal; and

selecting, from the plurality of time series of simulated rise of blood glucose level due to the meal, a time series of simulated rise of blood glucose level due to the meal that best matches the time series of estimated rise of blood glucose level due to the meal,

wherein the meal-specific values of the parameters of the physiological model are set based on a set of values of the parameters of the physiological model used to generate the selected time series of simulated rise of blood glucose level due to the meal.

**Clause 17.** The system of Clause 16, wherein determining the time series of estimated rise of blood glucose level due to the meal comprises:

estimating a time series of reduction of blood glucose level due to active insulin; and

determining the time series of estimated rise of blood glucose level due to the meal by adding the time series of reduction of blood glucose level due to active insulin to the portion of the measured glucose values.

**Clause 18.** One or more non-transitory processor-readable media storing instructions which, when executed by one or more processors, cause performance of operations comprising:

> obtaining measured glucose values of a person;
> fitting a physiological model to a portion of the measured glucose values within a time window after a start of a meal to determine meal-specific values of the parameters of the physiological model that characterizes the person's glycemic response to the meal; and
> predicting, using the physiological model and the meal-specific values of the parameters of the physiological model, a future blood glucose level of the person at a first time after the time window.

**Clause 19.** The one or more non-transitory processor-readable media of Clause 18, wherein predicting the future blood glucose level of the person comprises:

> estimating a reduction of glucose level by active insulin used up to the first time;
> generating, using the physiological model and the meal-specific values of the parameters of the physiological model, a predicted rise of blood glucose level due to the meal at the first time; and
> predicting the future blood glucose level of the person at the first time based on the reduction of glucose level by the active insulin used up to the first time and the predicted rise of blood glucose level due to the meal at the first time.

**Clause 20.** The one or more non-transitory processor-readable media of Clause 18 or 19, wherein fitting the physiological model to the portion of the measured glucose values comprises:

> determining, based on the measured glucose values, a time series of estimated rise of blood glucose level due to the meal;
> generating, using the physiological model and a plurality of sets of values of the parameters of the physiological model, a plurality of time series of simulated rise of blood glucose level due to the meal; and
> selecting, from the plurality of time series of simulated rise of blood glucose level due to the meal, a time series of simulated rise of blood glucose level due to the meal that best matches the time series of estimated rise of blood glucose level due to the meal,
> wherein the meal-specific values of the parameters of the physiological model are set based on a set of values of the parameters of the physiological model used to generate the selected time series of simulated rise of blood glucose level due to the meal.

Further disclosed herein is the subject-matter of the following items:

1. A processor-implemented method comprising:

> obtaining measured glucose values of a person;
> fitting a physiological model to a portion of the measured glucose values within a time window after a start of a meal to determine meal-specific values of parameters of the physiological model that characterizes the person's glycemic response to the meal; and
> predicting, using the physiological model and the meal-specific values of the parameters of the physiological model, a future blood glucose level of the person at a first time after the time window.

2. The processor-implemented method of item 1, wherein predicting the future blood glucose level of the person comprises:

> estimating a reduction of glucose level by active insulin used up to the first time;
> generating, using the physiological model and the meal-specific values of the parameters of the physiological model, a predicted rise of blood glucose level due to the meal at the first time; and
> predicting the future blood glucose level of the person at the first time based on the reduction of glucose level by the active insulin used up to the first time and the predicted rise of blood glucose level due to the meal at the first time.

3. The processor-implemented method of item 1 or 2, wherein the active insulin includes a meal bolus, basal insulin, a correction bolus, or a combination thereof.

4. The processor-implemented method of item 1 or of one of items 1 to 3, further comprising:

> determining a dose of a correction bolus; and
> causing delivery of the correction bolus to the person.

5. The processor-implemented method of item 1 or of one of items 1 to 4, further comprising detecting the start of the meal based on the measured glucose values.

6. The processor-implemented method of item 1 or of one of items 1 to 5, further comprising sending an alert or a notification to a user or an electronic device based on the predicted future blood glucose level of the person.

7. The processor-implemented method of item 1 or of one of items 1 to 6, wherein the parameters of the physiological model characterize a carbohydrate-to-glucose conversion factor and a rate of glucose absorption.

8. The processor-implemented method of item 1 or of one of items 1 to 7, wherein fitting the physiological model to the portion of the measured glucose values comprises:

> determining, based on the measured glucose values, a time series of estimated rise of blood glucose level due to the meal;
> generating, using the physiological model and a plurality of sets of values of the parameters of the physiological model, a plurality of time series of simulated rise of blood glucose level due to the meal; and
> selecting, from the plurality of time series of simulated rise of blood glucose level due to the meal, a time series of simulated rise of blood glucose level due to the meal that best matches the time series of estimated rise of blood glucose level due to the meal,
> wherein the meal-specific values of the parameters of the physiological model are set based on a set of values of the parameters of the physiological model used to generate the selected time series of simulated rise of blood glucose level due to the meal.

9. The processor-implemented method of item 8 or of one of items 1 to 8, wherein the selected time series of simulated rise of blood glucose level due to the meal has the lowest error with respect to the time series of estimated rise of blood glucose level due to the meal among the plurality of time series of simulated rise of blood glucose level due to the meal.

10. The processor-implemented method of item 8 or of one of items 1 to 9, wherein determining the time series of estimated rise of blood glucose level due to the meal comprises:

> estimating a time series of reduction of blood glucose level due to active insulin; and
> determining the time series of estimated rise of blood glucose level due to the meal by adding the time series of reduction of blood glucose level due to active insulin to the portion of the measured glucose values.

11. The processor-implemented method of item 1 or of one of items 1 to 10, wherein fitting the physiological model to the portion of the measured glucose values is based on a matching between:

> an estimated rate of appearance of glucose in blood determined based on the measured glucose values; and
> a simulated rate of appearance of glucose in blood determined using the physiological model.

12. The processor-implemented method of item 1 or of one of items 1 to 11, further comprising:

> predicting, for each candidate dose of a plurality of candidate doses of a correction bolus, future blood glucose levels of the person using the physiological model and the meal-specific values of the parameters of the physiological model; and
> selecting, from the plurality of candidate doses, a candidate dose as the dose of the correction bolus to be delivered to the person based on the predicted future blood glucose levels of the person for each candidate dose of the plurality of candidate doses of the correction bolus.

13. A system comprising:

> one or more processors; and
> one or more processor-readable media storing instructions which, when executed by the one or more processors, cause performance of operations including:

obtaining measured glucose values of a person;

fitting a physiological model to a portion of the measured glucose values within a time window after a start of a meal to determine meal-specific values of parameters of the physiological model that characterizes the person's glycemic response to the meal; and

predicting, using the physiological model and the meal-specific values of the parameters of the physiological model, a future blood glucose level of the person at a first time after the time window.

14. The system of item 13, wherein predicting the future blood glucose level of the person comprises:

estimating a reduction of glucose level by active insulin used up to the first time;

generating, using the physiological model and the meal-specific values of the parameters of the physiological model, a predicted rise of blood glucose level due to the meal at the first time; and

predicting the future blood glucose level of the person at the first time based on the reduction of glucose level by the active insulin used up to the first time and the predicted rise of blood glucose level due to the meal at the first time.

15. The system of item 13 or 14, wherein:

the physiological model includes a mathematical model or a machine-learning model for glycemic response prediction; and

the parameters of the physiological model characterize a carbohydrate-to-glucose conversion factor and a rate of glucose absorption.

16. The system of item 13 or of one of items 13 to 15, wherein fitting the physiological model to the portion of the measured glucose values comprises:

determining, based on the measured glucose values, a time series of estimated rise of blood glucose level due to the meal;

generating, using the physiological model and a plurality of sets of values of the parameters of the physiological model, a plurality of time series of simulated rise of blood glucose level due to the meal; and

selecting, from the plurality of time series of simulated rise of blood glucose level due to the meal, a time series of simulated rise of blood glucose level due to the meal that best matches the time series of estimated rise of blood glucose level due to the meal,

wherein the meal-specific values of the parameters of the physiological model are set based on a set of values of the parameters of the physiological model used to generate the selected time series of simulated rise of blood glucose level due to the meal.

17. The system of item 16 or of one of items 13 to 16, wherein determining the time series of estimated rise of blood glucose level due to the meal comprises:

estimating a time series of reduction of blood glucose level due to active insulin; and

determining the time series of estimated rise of blood glucose level due to the meal by adding the time series of reduction of blood glucose level due to active insulin to the portion of the measured glucose values.

18. One or more non-transitory processor-readable media storing instructions which, when executed by one or more processors, cause performance of operations comprising:

obtaining measured glucose values of a person;

fitting a physiological model to a portion of the measured glucose values within a time window after a start of a meal to determine meal-specific values of parameters of the physiological model that characterizes the person's glycemic response to the meal; and

predicting, using the physiological model and the meal-specific values of the parameters of the physiological model, a future blood glucose level of the person at a first time after the time window.

19. The one or more non-transitory processor-readable media of item 18, wherein predicting the future blood glucose level of the person comprises:

estimating a reduction of glucose level by active insulin used up to the first time;

generating, using the physiological model and the meal-specific values of the parameters of the physiological model, a predicted rise of blood glucose level due to the meal at the first time; and

predicting the future blood glucose level of the person at the first time based on the reduction of glucose level by the active insulin used up to the first time and the predicted rise of blood glucose level due to the meal at the first time.

20. The one or more non-transitory processor-readable media of item 18 or 19, wherein fitting the physiological model to the portion of the measured glucose values comprises:

determining, based on the measured glucose values, a time series of estimated rise of blood glucose level due to the meal;

generating, using the physiological model and a plurality of sets of values of the parameters of the physiological model, a plurality of time series of simulated rise of blood glucose level due to the meal; and

selecting, from the plurality of time series of simulated rise of blood glucose level due to the meal, a time series of simulated rise of blood glucose level due to the meal that best matches the time series of estimated rise of blood glucose level due to the meal,

wherein the meal-specific values of the parameters of the physiological model are set based on a set of values of the parameters of the physiological model used to generate the selected time series of simulated rise of blood glucose level due to the meal.

**Claims**

1. A processor-implemented method comprising:

obtaining measured glucose values of a person;

fitting a physiological model to a portion of the measured glucose values within a time window after a start of a meal to determine meal-specific values of parameters of the physiological model that characterizes the person's glycemic response to the meal; and

predicting, using the physiological model and the meal-specific values of the parameters of the physiological model, a future blood glucose level of the person at a first time after the time window.

2. The processor-implemented method of claim 1, wherein predicting the future blood glucose level of the person comprises:

estimating a reduction of glucose level by active insulin used up to the first time;

generating, using the physiological model and the meal-specific values of the parameters of the physiological model, a predicted rise of blood glucose level due to the meal at the first time; and

predicting the future blood glucose level of the person at the first time based on the reduction of glucose level by the active insulin used up to the first time and the predicted rise of blood glucose level due to the meal at the first time,

particularly

wherein the active insulin includes a meal bolus, basal insulin, a correction bolus, or a combination thereof.

3. The processor-implemented method of claim 1 or 2, further comprising:

determining a dose of a correction bolus; and

causing delivery of the correction bolus to the person.

4. The processor-implemented method of one of claims 1 to 3, further comprising detecting the start of the meal based on the measured glucose values,

and/or

further comprising sending an alert or a notification to a user or an electronic device based on the predicted future blood glucose level of the person.

5. The processor-implemented method of one of claims 1 to 4, wherein the parameters of the physiological model characterize a carbohydrate-to-glucose conversion factor and a rate of glucose absorption.

6. The processor-implemented method of one of claims 1 to 5, wherein fitting the physiological model to the portion of the measured glucose values comprises:

determining, based on the measured glucose values, a time series of estimated rise of blood glucose level due to the meal;
generating, using the physiological model and a plurality of sets of values of the parameters of the physiological model, a plurality of time series of simulated rise of blood glucose level due to the meal; and
selecting, from the plurality of time series of simulated rise of blood glucose level due to the meal, a time series of simulated rise of blood glucose level due to the meal that best matches the time series of estimated rise of blood glucose level due to the meal,
wherein the meal-specific values of the parameters of the physiological model are set based on a set of values of the parameters of the physiological model used to generate the selected time series of simulated rise of blood glucose level due to the meal.

7. The processor-implemented method of claim 6, wherein the selected time series of simulated rise of blood glucose level due to the meal has the lowest error with respect to the time series of estimated rise of blood glucose level due to the meal among the plurality of time series of simulated rise of blood glucose level due to the meal,
and/or
wherein determining the time series of estimated rise of blood glucose level due to the meal comprises:

estimating a time series of reduction of blood glucose level due to active insulin; and
determining the time series of estimated rise of blood glucose level due to the meal by adding the time series of reduction of blood glucose level due to active insulin to the portion of the measured glucose values.

8. The processor-implemented method of one of claims 1 to 7, wherein fitting the physiological model to the portion of the measured glucose values is based on a matching between:

an estimated rate of appearance of glucose in blood determined based on the measured glucose values; and
a simulated rate of appearance of glucose in blood determined using the physiological model.

9. The processor-implemented method of one of claims 1 to 8, further comprising:

predicting, for each candidate dose of a plurality of candidate doses of a correction bolus, future blood glucose levels of the person using the physiological model and the meal-specific values of the parameters of the physiological model; and
selecting, from the plurality of candidate doses, a candidate dose as the dose of the correction bolus to be delivered to the person based on the predicted future blood glucose levels of the person for each candidate dose of the plurality of candidate doses of the correction bolus.

10. A system comprising:

one or more processors; and
one or more processor-readable media storing instructions which, when executed by the one or more processors, cause performance of operations including:

obtaining measured glucose values of a person;
fitting a physiological model to a portion of the measured glucose values within a time window after a start of a meal to determine meal-specific values of parameters of the physiological model that characterizes the person's glycemic response to the meal; and
predicting, using the physiological model and the meal-specific values of the parameters of the physiological model, a future blood glucose level of the person at a first time after the time window.

11. The system of claim 10, wherein predicting the future blood glucose level of the person comprises:

estimating a reduction of glucose level by active insulin used up to the first time;
generating, using the physiological model and the meal-specific values of the parameters of the physiological model, a predicted rise of blood glucose level due to the meal at the first time; and
predicting the future blood glucose level of the person at the first time based on the reduction of glucose level by the active insulin used up to the first time and the predicted rise of blood glucose level due to the meal at the first time.

12. The system of claim 10 or 11, wherein:

the physiological model includes a mathematical model or a machine-learning model for glycemic response prediction; and
the parameters of the physiological model characterize a carbohydrate-to-glucose conversion factor and a rate of glucose absorption.

13. The system of one of claims 10 to 12, wherein fitting the physiological model to the portion of the measured glucose values comprises:

determining, based on the measured glucose values, a time series of estimated rise of blood glucose level due to the meal;
generating, using the physiological model and a plurality of sets of values of the parameters of the physiological model, a plurality of time series of simulated rise of blood glucose level due to the meal; and
selecting, from the plurality of time series of simulated rise of blood glucose level due to the meal, a time series of simulated rise of blood glucose level due to the meal that best matches the time series of estimated rise of blood glucose level due to the meal,
wherein the meal-specific values of the parameters of the physiological model are set based on a set of values of the parameters of the physiological model used to generate the selected time series of simulated rise of blood glucose level due to the meal,
particularly
wherein determining the time series of estimated rise of blood glucose level due to the meal comprises:

estimating a time series of reduction of blood glucose level due to active insulin; and
determining the time series of estimated rise of blood glucose level due to the meal by adding the time series of reduction of blood glucose level due to active insulin to the portion of the measured glucose values.

14. One or more non-transitory processor-readable media storing instructions which, when executed by one or more processors, cause performance of operations comprising:

obtaining measured glucose values of a person;
fitting a physiological model to a portion of the measured glucose values within a time window after a start of a meal to determine meal-specific values of parameters of the physiological model that characterizes the person's glycemic response to the meal; and
predicting, using the physiological model and the meal-specific values of the parameters of the physiological model, a future blood glucose level of the person at a first time after the time window.

15. The one or more non-transitory processor-readable media of claim 14, wherein predicting the future blood glucose level of the person comprises:

estimating a reduction of glucose level by active insulin used up to the first time;
generating, using the physiological model and the meal-specific values of the parameters of the physiological model, a predicted rise of blood glucose level due to the meal at the first time; and
predicting the future blood glucose level of the person at the first time based on the reduction of glucose level by the active insulin used up to the first time and the predicted rise of blood glucose level due to the meal at the first time,
and/or
wherein fitting the physiological model to the portion of the measured glucose values comprises:

determining, based on the measured glucose values, a time series of estimated rise of blood glucose level due to the meal;
generating, using the physiological model and a plurality of sets of values of the parameters of the physiological model, a plurality of time series of simulated rise of blood glucose level due to the meal; and
selecting, from the plurality of time series of simulated rise of blood glucose level due to the meal, a time series of simulated rise of blood glucose level due to the meal that best matches the time series of estimated rise of blood glucose level due to the meal,
wherein the meal-specific values of the parameters of the physiological model are set based on a set of values of the parameters of the physiological model used to generate the selected time series of simulated rise of blood glucose level due to the meal.

**FIG. 1A**

**FIG. 1B**

**FIG. 1C**

**FIG. 2**

**FIG. 3**

EP 4 679 440 A1

400

SG

410

Meal
420

430
insulin

414

412

432

insulin

Time

*FIG. 4*

EP 4 679 440 A1

**FIG. 5**

EP 4 679 440 A1

**FIG. 6**

700

Determine the rise of glucose level due to a meal based on measured glucose values
710

Set initial values of model parameters of a physiological model
720

Generate simulated glucose values using the physiological model and values of the model parameters
730

Tune values of model parameters
734

Calculate error (e.g.,: mean squared error) between the simulated glucose values and measured glucose values
732

Select values of model parameters that minimize the error
740

FIG. 7

**FIG. 8**

EP 4 679 440 A1

*FIG. 9*

**FIG. 10**

EP 4 679 440 A1

**FIG. 11**

**FIG. 12**

1300

```
┌─────────────────────────────────────────────────────────────────┐
│                  Obtain measured glucose values                   │
│                             1310                                  │
└─────────────────────────────────────────────────────────────────┘
                                 │
                                 ▼
┌─────────────────────────────────────────────────────────────────┐
│  Fit a physiological model to a portion of the measured glucose   │
│  values within a time window after a start of a meal to determine │
│         parameters of the physiological model                     │
│                             1320                                  │
└─────────────────────────────────────────────────────────────────┘
                                 │
                                 ▼
```

┌──────────────────────────────────────────────────────────────────────┐
│  ┌ ─ ─ ─ ─ ─ ─ ─ ─ ─ ─ ─ ─ ─ ─ ─ ─ ─ ─ ─ ─ ─ ─ ─ ─ ─ ─ ─ ─ ┐      │
│  │ Estimate the total amount of blood glucose increase due to the │      │
│  │                 meal based on the physiological model          │      │
│  │                           1332                                 │      │
│  └ ─ ─ ─ ─ ─ ─ ─ ─ ─ ─ ─ ─ ─ ─ ─ ─ ─ ─ ─ ─ ─ ─ ─ ─ ─ ─ ─ ┘      │
│                              │                                      │    1330
│                              ▼                                      │
│  ┌ ─ ─ ─ ─ ─ ─ ─ ─ ─ ─ ─ ─ ─ ─ ─ ─ ─ ─ ─ ─ ─ ─ ─ ─ ─ ─ ─ ┐      │
│  │     Estimate the total amount of glucose reduction by active   │      │
│  │                          insulin                               │      │
│  │                           1334                                 │      │
│  └ ─ ─ ─ ─ ─ ─ ─ ─ ─ ─ ─ ─ ─ ─ ─ ─ ─ ─ ─ ─ ─ ─ ─ ─ ─ ─ ─ ┘      │
│                              │                                      │
│                              ▼                                      │
│  ┌──────────────────────────────────────────────────────────┐      │
│  │            Estimate an amount of residual glucose          │      │
│  │                           1336                             │      │
│  └──────────────────────────────────────────────────────────┘      │
│                              │                                      │
│                              ▼                                      │
│  ┌ ─ ─ ─ ─ ─ ─ ─ ─ ─ ─ ─ ─ ─ ─ ─ ─ ─ ─ ─ ─ ─ ─ ─ ─ ─ ─ ─ ┐      │
│  │       Determine an amount of residual carbohydrates           │      │
│  │                           1338                                │      │
│  └ ─ ─ ─ ─ ─ ─ ─ ─ ─ ─ ─ ─ ─ ─ ─ ─ ─ ─ ─ ─ ─ ─ ─ ─ ─ ─ ─ ┘      │
└──────────────────────────────────────────────────────────────────────┘
                                 │
                                 ▼
┌─────────────────────────────────────────────────────────────────┐
│  Determine a dose of a correction bolus based on the residual     │
│              glucose or residual carbohydrates                    │
│                             1340                                  │
└─────────────────────────────────────────────────────────────────┘

*FIG. 13*

1400

1450

1470

1460

1440

1430

1432

1420

1410

*FIG. 14*

*FIG. 15*

EP 4 679 440 A1

**FIG. 16**

1700

```
┌────────────────────────────────────────────────────────┐
│           Obtain measured glucose data of a person      │
│                         1710                            │
└────────────────────────────────────────────────────────┘
                            │
                            ▼
┌ ─ ─ ─ ─ ─ ─ ─ ─ ─ ─ ─ ─ ─ ─ ─ ─ ─ ─ ─ ─ ─ ─ ─ ─ ─ ─ ─┐
│         Detect a start of a meal taken by the person    │
│                         1720                            │
└ ─ ─ ─ ─ ─ ─ ─ ─ ─ ─ ─ ─ ─ ─ ─ ─ ─ ─ ─ ─ ─ ─ ─ ─ ─ ─ ─┘
                            │
                            ▼
┌────────────────────────────────────────────────────────┐
│  Fit a physiological model to glucose data measured     │
│  within a time window after the start of the meal to    │
│  estimate parameters of the physiological model that    │
│  describes patient's response to the meal               │
│                         1730                            │
└────────────────────────────────────────────────────────┘
                            │
                            ▼
┌ ─ ─ ─ ─ ─ ─ ─ ─ ─ ─ ─ ─ ─ ─ ─ ─ ─ ─ ─ ─ ─ ─ ─ ─ ─ ─ ─┐
│              Determine a correction bolus dose          │◄ ─ ┐
│                         1740                            │    │
└ ─ ─ ─ ─ ─ ─ ─ ─ ─ ─ ─ ─ ─ ─ ─ ─ ─ ─ ─ ─ ─ ─ ─ ─ ─ ─ ─┘    │
                            │                                  │
                            ▼                                  │
┌ ─ ─ ─ ─ ─ ─ ─ ─ ─ ─ ─ ─ ─ ─ ─ ─ ─ ─ ─ ─ ─ ─ ─ ─ ─ ─ ─┐    │
│  Estimate glucose level drop caused by the correction   │    │
│  bolus and active insulin                               │    │
│                         1750                            │    │
└ ─ ─ ─ ─ ─ ─ ─ ─ ─ ─ ─ ─ ─ ─ ─ ─ ─ ─ ─ ─ ─ ─ ─ ─ ─ ─ ─┘    │
                            │                                  │
                            ▼                                  │
┌────────────────────────────────────────────────────────┐    │
│  Predict future glucose levels using the physiological  │    │
│  model with the estimated parameters (and glucose drop  │ ─ ─┘
│  caused by active insulin and the correction bolus)     │
│                         1760                            │
└────────────────────────────────────────────────────────┘
```

*FIG. 17*

**FIG. 18**

Europäisches
Patentamt

European
Patent Office

Office européen
des brevets

# EUROPEAN SEARCH REPORT

**Application Number**

EP 25 18 6993

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2023/000447 A1 (MIKHNO ARTHUR [US] ET AL) 5 January 2023 (2023-01-05)<br>* paragraph [0065] *<br>* paragraph [0067] - paragraph [0072] *<br>* paragraph [0101] *<br>* paragraph [0038] *<br>* figure 5 *<br>----- | 1-8,<br>10-15<br>9 | INV.<br>G16H20/17<br>G16H50/50<br>A61B5/145<br>A61B5/00<br>A61M5/172 |
| Y | EP 4 047 611 A1 (MEDTRONIC MINIMED INC [US]) 24 August 2022 (2022-08-24)<br>* paragraph [0067] - paragraph [0069] *<br>----- | 9 | |
| A | US 2020/101221 A1 (LINTEREUR LOUIS J [US] ET AL) 2 April 2020 (2020-04-02)<br>* paragraph [0005] - paragraph [0007] *<br>* paragraph [0080] - paragraph [0089] *<br>* figure 10 *<br>----- | 1-15 | |

**TECHNICAL FIELDS SEARCHED (IPC)**

G16H
A61B
A61M

The present search report has been drawn up for all claims

| Place of search | Date of completion of the search | Examiner |
|---|---|---|
| Munich | 3 November 2025 | Hauber, Jörg |

EPO FORM 1503 03.82 (P04C01)

## ANNEX TO THE EUROPEAN SEARCH REPORT
## ON EUROPEAN PATENT APPLICATION NO.

EP 25 18 6993

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2025

| Patent document cited in search report | Publication date | Patent family member(s) | Publication date |
|---|---|---|---|
| US 2023000447 A1 | 05-01-2023 | EP 4364157 A1 | 08-05-2024 |
| | | US 2023000447 A1 | 05-01-2023 |
| | | WO 2023278653 A1 | 05-01-2023 |
| EP 4047611 A1 | 24-08-2022 | CN 114949444 A | 30-08-2022 |
| | | EP 4047611 A1 | 24-08-2022 |
| | | US 2022265177 A1 | 25-08-2022 |
| | | US 2025281077 A1 | 11-09-2025 |
| US 2020101221 A1 | 02-04-2020 | AU 2019346813 A1 | 11-03-2021 |
| | | CA 3110085 A1 | 02-04-2020 |
| | | CN 111246901 A | 05-06-2020 |
| | | EP 3856286 A1 | 04-08-2021 |
| | | JP 2022502158 A | 11-01-2022 |
| | | KR 20210068404 A | 09-06-2021 |
| | | US 2020101221 A1 | 02-04-2020 |
| | | US 2021228805 A1 | 29-07-2021 |
| | | US 2023302221 A1 | 28-09-2023 |
| | | WO 2020068191 A1 | 02-04-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63669067

- US 63669069

**Non-patent literature cited in the description**

- **GROSMAN et al.** Sensor-augmented pump-based customized mathematical model for Type 1 diabetes. *DIABETES TECHNOLOGY & THERAPEUTICS*, 2018, vol. 20 (3), 207-221

- **GROSMAN et al.** Fast-acting insulin aspart (Fiasp®) improves glycemic outcomes when used with Mini-Med™ 670G hybrid closed-loop system in simulated trials compared to NovoLog. *Computer Methods and Programs in Biomedicine*, 2021, vol. 205